# EUROPÄISCHE PATENTANMELDUNG

(11) **EP 4 553 456 A1**
(43) Veröffentlichungstag der Anmeldung: **14.05.2025**
(21) Anmeldenummer: 24210547.6
(22) Anmeldetag: 04.11.2024
(51) Int. Cl.: G01D 21/00, G01D 4/00

(54) **VERFAHREN UND VORRICHTUNG ZUM BETREIBEN EINER SENSOR-ANORDNUNG**

(30) Priorität: 07.11.2023 DE 102023130701
(71) Anmelder: Dräger Safety AG & Co. KGaA, 23560 Lübeck (DE)
(72) Erfinder: Rodehorst, Christof, 23558 Lübeck (DE); Furuta, Jun, 23558 Lübeck (DE); Einecke, Kai, 23558 Lübeck (DE)

(57) **Zusammenfassung**

Die Erfindung betrifft ein Betriebs-Verfahren und eine Betriebs- Anordnung, um eine Sensor-Anordnung zu betreiben. Diese Sensor-Anordnung umfasst mindestens einen Sensor (1.2), bevorzugt mehrere Sensoren. Für jeden Sensor wird eine Installationsphase durchgeführt. Ein Installationsgerät erzeugt mindestens ein Referenzbild von einem Installationsort (lo.2), an dem der Sensor installiert ist. In einer zentralen Datenbank wird ein Datensatz für den Sensor angelegt, wobei dieser Datensatz das oder jedes Referenzbild umfasst. Für mindestens ein Sensor (1.2) wird eine nachfolgende Suchphase durchgeführt. Ein Suchgerät (4) erzeugt mindestens ein Suchbild (31.1, ..., 31.4) von der Umgebung des Suchgeräts. Das oder jede Suchbild wird mit dem oder jedem abgespeicherten Referenzbild verglichen. Das Ergebnis des Bildvergleichs wird an das Suchgerät (4) übermittelt. Das Suchgerät (4) ermittelt eine Information (41.2) über den Installationsort (lo.2) des Sensors (1.2), wofür es das Ergebnis des Bildvergleichs verwendet. Das Suchgerät gibt die Installationsort-information (41.2) in einer von einem Menschen wahrnehmbaren Form aus.

## Beschreibung

Die Erfindung betrifft ein Betriebs-Verfahren und eine Betriebs-Anordnung, um eine Sensor-Anordnung zu betreiben. Diese Sensor-Anordnung umfasst mindestens einen Sensor, bevorzugt mehrere Sensoren. Weiterhin betrifft die Erfindung ein System mit einer solchen Sensor-Anordnung und einer solchen Betriebs-Anordnung.

Ein Beispiel für einen solchen Sensor ist ein Gasmessgerät. In einer Anwendung vermag ein Gasmessgerät die Konzentration eines für Menschen schädlichen oder auch notwendigen Zielgases zu messen und in einer Ausgestaltung einen Alarm zu generieren, wenn die Zielgas-Konzentration oberhalb oder auch unterhalb einer vorgegebenen Konzentrations-Schranke liegt. Das Zielgas ist insbesondere ein brennbares oder toxisches Gas, kann aber z.B. auch Sauerstoff, Kohlendioxid oder ein Narkosemittel sein.

Zwei mögliche Arten, um ein solches Gasmessgerät zu verwenden, sind die folgenden:
- mobiles (tragbares) Gasmessgerät: Ein Benutzer führt das Gasmessgerät mit sich, während er sich in einem Bereich aufhält, in dem ein Zielgas auftritt oder wenigstens auftreten kann. Das Gasmessgerät gibt die Zielgas-Konzentration aus und / oder warnt den Benutzer, falls es eine Zielgas-Konzentration oberhalb oder auch unterhalb (Sauerstoff) der Konzentrations-Schranke misst.
- stationäres (ortsfestes) Gasmessgerät: Das Gasmessgerät wird an einem Ort installiert, wobei an diesem Ort mindestens ein Zielgas auftritt oder wenigstens auftreten kann, und verbleibt während eines Einsatzes an diesem Ort. Das stationäre Gasmessgerät übermittelt Alarme und / oder gemessene Zielgas-Konzentrationen an mindestens einen räumlich entfernten Empfänger.

Diese Unterscheidung zwischen mobil und stationär gilt entsprechend auch für andere Sensoren.

Die Erfindung bezieht sich auf eine Sensor-Anordnung mit stationären Sensoren, im Falle von Gasmessgeräten als den Sensoren also auf die zweite Anwendung. Die meiste Zeit wird kein Mensch benötigt, um einen stationären Sensor zu betreiben. Jedoch muss in vielen Fällen ein Servicetechniker den Sensor regelmäßig überprüfen und bei Bedarf kalibrieren und / oder reparieren.

Der Erfindung liegt die Aufgabe zugrunde, ein Betriebs-Verfahren und eine Betriebs-Anordnung bereitzustellen, um eine Sensor-Anordnung mit mindestens einem stationären Sensor, bevorzugt mit mehreren stationären Sensoren, zu betreiben, wobei das Betriebs-Verfahren und die Betriebs-Anordnung weniger Aufwand als bekannte Betriebs-Verfahren und Betriebs-Anordnungen erfordern sollen.

Die Aufgabe wird durch ein Betriebs-Verfahren mit den Merkmalen des Anspruchs 1 und durch eine Betriebs-Anordnung mit den Merkmalen des Anspruchs 18 gelöst. Vorteilhafte Ausgestaltungen des erfindungsgemäßen Betriebs-Verfahrens sind, soweit sinnvoll, auch vorteilhafte Ausgestaltungen der erfindungsgemäßen Betriebs-Anordnung und umgekehrt.

Das erfindungsgemäße Betriebs-Verfahren und die erfindungsgemäße Betriebs-Anordnung ermöglichen es, eine Sensor-Anordnung zu betreiben. Die Sensor-Anordnung umfasst mindestens einen Sensor, bevorzugt mehrere Sensoren. Der oder jeder Sensor der Sensor-Anordnung vermag in einer ersten Alternative mindestens eine physikalische Größe zu messen, optional mehrere Größen. Wenn nachfolgend davon gesprochen wird, dass ein Sensor eine physikalische Größe misst, so ist damit folgendes gemeint: Der Sensor misst mindestens einmal, welchen Wert diese physikalische Größe oder eine andere physikalische Größe zum jeweiligen Messzeitpunkt annimmt. Die andere physikalische Größe korreliert mit der zu messenden physikalischen Größe, ist also ein Maß für die zu messende Größe. In einer zweiten Alternative vermag der oder jeder Sensor automatisch zu entscheiden, ob die jeweilige physikalische Größe in einem vorgegebenen Wertebereich liegt oder nicht. Bevorzugt bedeutet dies folgendes: Der Sensor entscheidet, ob der Wert der Größe in einem Bezugs-Zeitraum durchgehend im Wertebereich oder wenigstens zeitweise außerhalb des Wertebereichs liegt, wobei der Bezugs-Zeitraum insbesondere ein gleitendes Zeitfenster oder Zeitpunkt ist.

Möglich ist, dass derselbe Sensor sowohl die Größe zu messen vermag als auch zu entscheiden vermag, ob die Größe in den vorgegebenen Wertebereich fällt oder nicht. Die Alternativen lassen sich auch wie folgt kombinieren: Mindestens ein erster Sensor vermag die physikalische Größe zu messen, und mindestens ein zweiter Sensor vermag automatisch zu entscheiden, ob die Größe in einen vorgegebenen Wertebereich fällt oder nicht.

Die gemessene physikalische Größe kann für jeden Sensor der Sensor-Anordnung dieselbe sein. Möglich ist auch, dass die Sensoren der Sensor-Anordnung insgesamt mindestens zwei verschiedene physikalische Größen zu messen vermögen. Möglich ist auch, dass mindestens ein Sensor der Sensor-Anordnung gleichzeitig mindestens zwei verschiedene physikalische Größen zu messen vermag.

Im Falle von Gasmessgeräten als den Sensoren vermag jeder Sensor jeweils mindestens ein vorgegebenes Zielgas zu detektieren und / oder die Konzentration mindestens eines Zielgases zu messen. Dass das Zielgas vorgegeben ist, bedeutet folgendes: Vorgegeben ist, auf welches Zielgas oder welche Zielgase ein räumlicher Bereich zu überwachen ist. Das Zielgas ist insbesondere ein brennbares oder giftiges oder auf andere Weise für Menschen schädliches Gas oder auch Sauerstoff oder ein anderes lebensnotwendiges Gas oder auch Kohlendioxid oder ein Narkosemittel. Die gesuchte Zielgas-Konzentration ist also die zu messende physikalische Größe, und ein zulässiger Bereich für die Zielgas-Konzentration ist als Wertebereich vorgegeben. Das zu detektierende Zielgas kann für jedes Gasmessgerät der Sensor-Anordnung dasselbe sein. Möglich ist auch, dass die Sensoren der Sensor-Anordnung insgesamt mindestens zwei verschiedene Zielgase zu detektieren und bevorzugt voneinander zu unterscheiden vermögen. Möglich ist, dass mindestens ein Sensor der Sensor-Anordnung als die Zielgas-Konzentration eine summierte Konzentration von mehreren gleichzeitig auftretenden Zielgasen zu messen vermag. In der Regel ist die physikalische Größe, für welche der Sensor einen Wert misst, eine Variable, die mit der Zielgas-Konzentration korreliert, insbesondere eine elektrische Spannung oder Stromstärke oder Leistung oder Ladung oder Temperatur.

Jeder Sensor der Sensor-Anordnung ist während eines Einsatz-Zeitraums an jeweils einem Installationsort installiert, ist also ein stationärer Sensor im Sinne der eingangs getroffenen Unterscheidung. Möglich ist, dass eine Person den stationären Sensor zum Installationsort zu tragen und später wieder von dort abzutransportieren vermag. In der Regel montiert jeweils eine Person jeden Sensor am jeweiligen Installationsort und nimmt den Sensor dort in Betrieb. Möglich ist auch, dass der stationäre Sensor für einen Einsatz auf einen Untergrund gestellt wird. Diese Person wird "der Installationstechniker" genannt. Natürlich ist es möglich, dass mehrere Installationstechniker am Vorgang beteiligt sind, die Sensoren der Sensor-Anordnung zu installieren. Der Vorgang, den oder jeden Sensor der Sensor-Anordnung am jeweiligen Installationsort zu installieren, wird als "die Installationsphase" bezeichnet.

In der Regel ist es beim Einsatz der Sensor-Anordnung erforderlich oder wenigstens sinnvoll, dass eine Person während des Einsatzes jeden Sensor am jeweiligen Installationsort überprüft, wartet, kalibriert und / oder repariert. Diese Person wird nachfolgend als "der Servicetechniker" bezeichnet. Der Servicetechniker muss dazu in der Regel mindestens einmal den Sensor am Installationsort auffinden, nämlich dann, wenn eine Wartung ausschließlich aus der Ferne nicht möglich ist, beispielsweise weil ein Teil des Sensors ausgetauscht werden muss, oder wenn keine Datenverbindung mit dem Sensor besteht. Letzteres ist in der Regel eine unerwünschte Situation, die meist vor Ort zu beseitigen ist. Der Sensor, der kalibriert oder gewartet oder repariert werden muss, wird im Folgenden als ein "aufzufindender Sensor" bezeichnet. Der Vorgang, mindestens einen Sensor der Sensor-Anordnung an seinem jeweiligen Installationsort aufzufinden, wird als "die Suchphase" bezeichnet. Natürlich ist möglich, dass verschiedene Servicetechniker für die Sensoren der Sensor-Anordnung eingesetzt werden. In der Praxis tritt häufig die Situation auf, dass der oder mindestens ein Installationstechniker und der oder mindestens ein Servicetechniker zwei verschiedene Personen sind.

Die erfindungsgemäße Betriebs-Anordnung umfasst ein mobiles datenverarbeitendes Installationsgerät, ein mobiles datenverarbeitendes Suchgerät, einen Zentralrechner und eine zentrale Datenbank. Das erfindungsgemäße Betriebs-Verfahren wird unter Verwendung einer solchen Betriebs-Anordnung durchgeführt.

Unter einem datenverarbeitenden Gerät wird ein Gerät verstanden, welches Daten und / oder Signale zu verarbeiten vermag und in der Regel einen Prozessor, eine Eingabeeinheit und eine Ausgabeeinheit umfasst. Mit dem Begriff "mobiles Gerät" wird ein Gerät bezeichnet, welches dazu ausgestaltet ist, von einem Menschen mit sich geführt zu werden, und in der Regel eine eigene Spannungsversorgungseinheit umfasst. Insbesondere sind ein Smartphone, ein Tablet oder ein sonstiger tragbarer Rechner jeweils ein mobiles und datenverarbeitendes Gerät im Sinne der Erfindung. Möglich ist, dass dasselbe Gerät sowohl als das Installationsgerät als auch als das Suchgerät fungiert. In dieser Realisierungsform sind bevorzugt auf diesem Gerät zwei verschiedene Programme installiert, nämlich ein Programm für die Schritte der Installationsphase und ein Programm für die Schritte der Suchphase. Möglich ist auch, dass zwei verschiedene Geräte als das Installationsgerät bzw. als das Suchgerät fungieren. Diese beiden Geräte können gleichartig oder unterschiedlich aufgebaut sein und verwenden bevorzugt zwei verschiedene Programme für die zwei verschiedenen Phasen.

In der Regel verwendet der Installationstechniker das Installationsgerät, der Servicetechniker das Suchgerät. Möglich ist natürlich, dass nacheinander verschiedene Installationstechniker dasselbe Installationsgerät und / oder verschiedene Servicetechniker nacheinander dasselbe Suchgerät verwenden. Möglich ist auch, dass dieselbe Person zunächst als Installationstechniker arbeitet und das Installationsgerät benutzt und anschließend dieselbe Person als Servicetechniker arbeitet und dasselbe oder ein anderes Gerät als das Suchgerät verwendet. Möglich ist auch, dass in der Installationsphase mindestens zwei verschiedene Installationsgeräte und / oder in der oder einer Suchphase mindestens zwei Suchgeräte verwendet werden. Nachfolgend wird abkürzend von dem Installationsgerät und dem Suchgerät gesprochen.

Der Zentralrechner und die zentrale Datenbank sind in der Regel als jeweils ein ortsfestes Gerät ausgestaltet, können aber auch auf einem tragbaren Rechner realisiert sein, beispielsweise auf einem Smartphone. Möglich ist insbesondere, dass der Zentralrechner und die zentrale Datenbank auf dem Installationsgerät und / oder auf dem Suchgerät realisiert sind. Der Zentralrechner und die zentrale Datenbank können auch als Bestandteile von jeweils einem Server im Internet realisiert sein, insbesondere "in der Cloud". Möglich ist, dass nacheinander jeweils ein Bereich von unterschiedlichen Servern verwendet wird, um den Zentralrechner und die zentrale Datenbank zu realisieren.

Wenigstens zeitweise ist eine Datenverbindung vom Installationsgerät zum Zentralrechner hergestellt. Diese Datenverbindung ist bevorzugt eine drahtlose Datenverbindung, also eine Datenverbindung per Funkwellen, insbesondere unter Verwendung eines öffentlichen Mobilfunknetzes oder auch eines Richtfunknetzes. Die Datenverbindung kann auch eine kabelgebundene Datenverbindung sein. Ausreichend ist eine unidirektionale Datenverbindung, eine bidirektionale Datenverbindung ist ebenfalls möglich.

Das Installationsgerät umfasst ein Bildaufnahmegerät, insbesondere eine Digitalkamera oder eine Videokamera. Das Suchgerät umfasst bevorzugt ebenfalls ein Bildaufnahmegerät. Das oder jedes Bildaufnahmegerät vermag jeweils mindestens ein Bild zu erzeugen, bevorzugt eine Abfolge von Bildern.

Das Betriebs-Verfahren umfasst eine Installationsphase und eine nachfolgende Suchphase. Die Installationsphase wird für jeden Sensor der Sensor-Anordnung durchgeführt. Während der Installationsphase benutzt der Installationstechniker das Installationsgerät. Die Suchphase wird für mindestens einen aufzufindenden Sensor der Sensor-Anordnung durchgeführt, also wenn der Servicetechniker den Sensor auffinden muss oder soll, um an diesem Sensor eine Handlung vorzunehmen. Während der Suchphase benutzt der Servicetechniker das Suchgerät. Möglich ist, dass eine Suchphase für mehrere oder sogar alle Sensoren der Sensor-Anordnung durchgeführt wird. Möglich ist auch, dass nacheinander mehrere Suchphasen für jeweils mindestens einen Sensor durchgeführt werden, auch von unterschiedlichen Servicetechnikern und / oder mit unterschiedlichen Suchgeräten und auch mehrmals für denselben Sensor.

Das Installationsgerät umfasst ein Bildaufnahmegerät, bevorzugt in Form einer Digitalkamera. Falls dasselbe mobile Gerät sowohl als das Installationsgerät als auch als das Suchgerät verwendet wird, so wird bevorzugt dasselbe Bildaufnahmegerät dieses Gerät sowohl in der Installationsphase als auch in der Suchphase verwendet.

Wie bereits erwähnt, wird die Installationsphase für jeden Sensor durchgeführt. Für jeden Sensor umfasst die Installationsphase jeweils die folgenden Schritte:
- Das Bildaufnahmegerät des Installationsgeräts erzeugt mindestens ein Referenzbild vom Installationsort des Sensors. Bevorzugt zeigt das oder mindestens ein Referenzbild den am Installationsort installierten Sensor. Möglich ist, dass mindestens ein weiteres Referenzbild einen Gegenstand in der Nähe des Sensors zeigt, insbesondere einen Gegenstand, der dauerhaft oder wenigstens in einem von mehreren möglichen Zuständen die Sicht auf den Sensor verdeckt, beispielsweise eine Tür. In der Regel verwendet der Installationstechniker das Bildaufnahmegerät des Installationsgeräts, um die Referenzbilder zu erzeugen.
- Das oder mindestens ein, bevorzugt jedes Referenzbild, das beim Installieren des Sensors am Installationsort erzeugt worden ist, wird vom Installationsgerät an den Zentralrechner übermittelt. Hierfür wird die oben beschriebene Datenverbindung verwendet.
- Der Zentralrechner bewirkt den folgenden Schritt: In der zentralen Datenbank wird ein Datensatz für den Sensor angelegt. Falls ein solcher Datensatz bereits angelegt ist, so wird dieser Datensatz so wie nachfolgend beschrieben ergänzt.
- Nach dem Anlegen oder Ergänzen umfasst der Datensatz das oder mindestens ein, bevorzugt jedes Referenzbild, welches am Installationsort aufgenommen und an den Zentralrechner übermittelt worden ist, optional mit jeweils einem Zeitstempel und / oder mit der jeweiligen Geoposition für jedes Referenzbild. Die Geoposition beschreibt bevorzugt den Ort, an dem das Installationsgerät das Referenzbild aufgenommen hat. In einer Realisierungsform wird am Installationsort eine eindeutige Kennung des Sensors erfasst und an den Zentralrechner übermittelt, und der Datensatz umfasst die erfasste und übermittelte eindeutige Kennung. Beispielsweise lässt sich die eindeutige Kennung erfassen, indem ein Referenzbild vom Sensor ausgelesen wird. Oder ein Lesegerät, z.B. ein NFC-Leser oder Barcode-Leser, liest die eindeutige Kennung des Sensors.

Die oder jede Suchphase wird für mindestens einen aufzufindenden Sensor durchgeführt. Die Suchphase umfasst für den oder jeden aufzufindenden Sensor jeweils die folgenden Schritte:
- Der Datensatz für den aufzufindenden Sensor in der zentralen Datenbank wird ermittelt. Das oder jedes Referenzbild wird ermittelt, welches von dem ermittelten Datensatz umfasst ist.
- Die Betriebs-Anordnung ermittelt eine Information über den Installationsort des aufzufindenden Sensors. Für diese Ermittlung verwendet die Betriebs-Anordnung das oder jedes ermittelte Referenzbild, optional weitere Informationen.
- Das Suchgerät gibt die ermittelte Installationsort-Information in mindestens einer von einem Menschen wahrnehmbaren Form aus. Die ausgegebene Installationsort-Information umfasst in einer Ausgestaltung das oder mindestens ein ermitteltes Referenzbild des aufzufindenden Sensors.

In einer Ausgestaltung ermittelt das Suchgerät selbst die Installationsort-Information für einen Sensor. In einer anderen Ausgestaltung ermittelt ein räumlich entferntes datenverarbeitendes Gerät die Installationsort-Information, und diese wird an das Suchgerät übermittelt.

Die Erfinder haben intern folgendes Problem identifiziert: Der Servicetechniker kennt sich häufig nicht genau auf einer Anlage oder sonstigem Gelände aus, auf der der aufzufindende Sensor eingesetzt wird. In vielen Fällen ist der Servicetechniker, der einen Sensor warten oder reparieren soll, nicht diejenige Person, die diesen Sensor installiert hat. Häufig vergeht relativ viel Zeit, bis ein stationärer Sensor beispielsweise auf einer Produktionsanlage oder in einer Lagerhalle oder in einem Gebäude oder an Bord eines Fahrzeugs aufgefunden wird. Der Sensor am Installationsort kann nämlich relativ schwer aufzufinden sein. In vielen Fällen ist außerdem der Installationsort, an dem der Sensor installiert ist, nicht ausreichend genau dokumentiert. Oder die Dokumentation steht bei der Suchphase nicht zur Verfügung oder ist veraltet oder nicht dafür geeignet, um von einem Servicetechniker transportiert zu werden. Die Erfindung stellt oft eine Lösung für dieses Problem bereit.

Wie bereits erwähnt, ist in vielen Fällen der oder ein Servicetechniker nicht zugleich der Installationstechniker. Dies ist insbesondere dann der Fall, wenn als Installationstechniker ein Techniker des Herstellers die Sensoren installiert und später als Servicetechniker ein Techniker eines Betreibers der Sensor-Anordnung einen Sensor auffinden soll oder muss. Daher benötigt häufig der Servicetechniker eine Unterstützung, um zum Installationsort geführt zu werden. Oft sind Beschreibungen oder sonstige Unterlagen über den Installationsort nicht vorhanden oder nicht verfügbar oder veraltet oder nicht genau genug. Die Erfindung lässt sich in Kombination mit solchen Unterlagen verwenden, setzt aber nicht voraus, dass eine Unterlage über den Installationsort verfügbar ist oder verfügbar gemacht wird.

Die Erfindung spart also Zeit ein, die ein Servicetechniker benötigt, um einen Sensor aufzufinden. In vielen Fällen hat der Servicetechniker dank der Erfindung mehr produktive Zeit, um den Sensor zu warten oder zu reparieren oder eine sonstige Arbeit am Sensor durchzuführen, oder kann in der gleichen Zeit mehr Sensoren aufsuchen oder benötigt weniger Zeit. Außerdem reduziert die Erfindung die Gefahr, dass ein Servicetechniker einen Sensor gar nicht oder erst nach langer Suchzeit findet oder aber einen falschen Sensor auffindet.

Die Erfindung lässt sich in Kombination mit einen Geopositionssensor verwenden, wobei der Geopositionssensor zum Suchgerät gehört und das Suchgerät mit Hilfe von gemessenen Geopositionen den Servicetechniker an die Geoposition des Installationsorts des Sensors führt. Bevorzugt gehört zum Datensatz für einen Sensor der Sensor-Anordnung die Geoposition des Installationsorts, wobei diese Geoposition in der Installationsphase gemessen und als Teil des Datensatzes für den Sensor abgespeichert worden ist. Geopositionssensoren als Bestandteile von Smartphones und anderen mobilen datenverarbeitenden Geräten sind wohlbekannt und weit verbreitet.

Jedoch lässt sich in manchen Fällen die Geoposition nicht ausreichend genau messen, insbesondere wenn der verwendete Geopositionssensor sich im Inneren eines Gebäudes oder Fahrzeugs befindet. Insbesondere in diesem Fall kann ein Signal eines Geopositionssensors mit Fehlern behaftet sein, beispielsweise weil ein Signal, welches der Geopositionssensor benötigt, abgeschirmt wird. Der Installationsort kann relativ verborgen sein, so dass der Sensor sich trotz einer relativ genau gemessenen Geoposition nur schwer auffinden lässt. Außerdem vermag ein Geopositionssensor häufig nicht oder nicht genau genug die Höhe eines Orts über NN oder über dem Erdboden zu messen. Diese Höhe ist aber insbesondere dann häufig wichtig, um einen Sensor aufzufinden, wenn der Sensor in einem mehrstöckigen Gebäude oder Fahrzeug eingesetzt wird, und besonders wichtig, wenn in diesem Gebäude oder Fahrzeug mehrere gleichartige Sensoren eingesetzt werden. Um den Servicetechniker mittels eines Geopositionssensors zum Installationsort zu führen, ist es außerdem erforderlich, in der Installationsphase die Geoposition des Installationsorts zu messen, abzuspeichern und verfügbar zu machen.

Die Erfindung zeigt einen Weg auf, wie der Servicetechniker den Installationsort zusätzlich zu den oder anstelle der Geoposition, die ein Geopositionssensor in der Installationsphase gemessen hat, auffindet, nämlich mit Hilfe der Referenzbilder. Die Erfindung vermeidet daher in vielen Fällen die Nachteile, die entstehen würden, wenn der Servicetechniker ausschließlich mit Hilfe eines Signals eines Geopositionssensors zum Installationsort geführt werden würde.

Erfindungsgemäß wird in der Suchphase für jeden Sensor jeweils eine Information über den Installationsort dieses Sensors ermittelt. Hierfür wird das oder jedes Referenzbild dieses Sensors ermittelt, wobei das oder jedes ermittelte Referenzbild zum Datensatz für diesen Sensor gehört. Das Suchgerät gibt die ermittelte Installationsort-Information in einer von einem Menschen wahrnehmbaren Form aus.

In einer Ausgestaltung gibt das Suchgerät einem Servicetechniker eine Information vor, welchen Sensor oder welche Sensoren der Servicetechniker in einer Suchphase aufsuchen soll, und bevorzugt eine Information, welche Arbeit an dem Sensor jeweils durchzuführen ist. Das Suchgerät vermag folgendes zu erfassen: eine Kennung des aufzufindenden Sensors und / oder eine Eingabe des Servicetechnikers, den aufzufindenden Sensor nunmehr gefunden zu haben, und / oder eine Eingabe des Servicetechnikers, die erforderliche Arbeit am Sensor durchgeführt zu haben. Die Kennung des aufzufindenden Sensors stammt beispielsweise aus einem Arbeitsplan für den Servicetechniker.

Bevorzugt erfasst das Suchgerät eine Eingabe, dass der aufzufindende Sensor nunmehr gefunden worden ist. Beispielsweise erfasst das Suchgerät eine entsprechende Bestätigung (Benutzereingabe) des Servicetechnikers. Möglich ist auch, dass das Suchgerät ein Lesegerät umfasst, wobei das Lesegerät eine eindeutige Kennung des Sensors auszulesen vermag. Die eindeutige Kennung ist beispielsweise in Form eines Barcodes auf ein Gehäuse des Sensors aufgebracht oder in einem NFC-Tag, insbesondere einem RFID-Tag, abgespeichert. Die eindeutige Kennung unterscheidet diesen Sensor mindestens von allen anderen Sensoren der Sensor-Anordnung. Bei der ersten Realisierungsform vermag das Lesegerät einen Barcode zu lesen, bei der zweiten Ausführungsform einen NFC-Tag auszulesen. Falls dieses Lesegerät die eindeutige Kennung des aufzufindenden Sensors ausgelesen hat, ist das Sensor aufgefunden. Möglich ist auch, dass als eindeutige Kennung eine alphanumerische Zeichenfolge und / oder ein Symbol dergestalt auf den Sensor aufgebracht ist, dass ein Mensch und / oder ein Lesegerät diese Kennung zu lesen oder dieses Symbol zu erkennen vermögen.

In einer Ausgestaltung umfasst die ermittelte Installationsort-Information das oder mindestens ein, bevorzugt jedes Referenzbild des Sensors, wobei dieses Referenzbild in der Installationsphase erzeugt wurde und zum Datensatz gehört. Das oder jedes ermittelte Referenzbild wird von der zentralen Datenbank an das Suchgerät übermittelt. Das Suchgerät gibt das oder mindestens ein, bevorzugt jedes übermittelte Referenzbild aus, und zwar als Bestandteil der ausgegebenen Installationsort-Information.

Diese Ausgestaltung lässt sich relativ einfach realisieren. Die ausgegebenen Referenzbilder erleichtern es einem Servicetechniker, den Installationsort aufzufinden. In einer Realisierungsform werden die Datensätze der Sensoren der Sensor-Anordnung auf dem Suchgerät installiert. In einer anderen Realisierungsform wird bei Bedarf der Datensatz für einen aufzufindenden Sensor -oder wenigstens ein Teil des Datensatzes - vom Zentralrechner an das Suchgerät übermittelt. Die Ausgestaltung mit den Referenzbildern und die Ausgestaltung mit den Geopositionen lassen sich miteinander kombinieren.

In einer bevorzugten Ausgestaltung umfasst die Betriebs-Anordnung eine signalverarbeitende Bildvergleichseinheit. Nicht nur das Installationsgerät, sondern auch das Suchgerät umfasst gemäß dieser bevorzugten Ausgestaltung ein Bildaufnahmegerät. Gemäß dieser Ausgestaltung umfasst die Suchphase für den oder jeden aufzufindenden Sensor jeweils die folgenden zusätzlichen Schritte:
- Das Bildaufnahmegerät des Suchgeräts erzeugt mindestens ein Suchbild von einer Umgebung des Suchgeräts, bevorzugt mehrere Suchbilder von der Umgebung. Bevorzugt verwendet der Servicetechniker das Bildaufnahmegerät des Suchgeräts, um die Suchbilder zu erzeugen.
- An die Bildvergleichseinheit wird einerseits das oder mindestens ein, bevorzugt jedes Referenzbild übermittelt. Das oder jedes übermittelte Referenzbild wurde in der Installationsphase aufgenommen, und zwar vom oder am Installationsort des Sensors, und gehört zum Datensatz für den Sensor.
- Andererseits wird an die Bildvergleichseinheit das oder mindestens ein, bevorzugt jedes Suchbild, das in der Suchphase aufgenommen wurde, übermittelt.
- Die Bildvergleichseinheit ermittelt in den übermittelten Suchbildern den Installationsort des Sensors. Hierfür führt die Bildvergleichseinheit einen rechnerischen Bildvergleich durch, bei dem mindestens ein Suchbild mit mindestens einem Referenzbild des aufzufindenden Sensors verglichen wird. Bevorzugt sucht die Bildvergleichseinheit in den übermittelten Suchbildern nach einem Abbild des aufzufindenden Sensors, wobei dieser Sensor in mindestens einem Referenzbild gezeigt wird. Falls vorhanden, vergleicht die Bildvergleichseinheit alphanumerische Kennungen oder Symbole oder Barcodes in den Referenzbildern mit denen in den Suchbildern.
- Das Ergebnis des Bildvergleichs wird an das Suchgerät übermittelt. Möglich ist auch, dass die Bildvergleichseinheit feststellt, dass kein Suchbild mit ausreichender Sicherheit den Installationsort zeigt.
- Das Suchgerät ermittelt eine Information über den Installationsort. Hierfür verwendet das Suchgerät die übermittelten Ergebnisse des Bildvergleichs.
- Das Suchgerät gibt die ermittelte Information über den Installationsort in mindestens einer Form aus, die ein Mensch wahrnehmen kann, insbesondere visuell.

Die beiden letzten Schritte werden natürlich nur dann durchgeführt, wenn die Bildvergleichseinheit in mindestens einem Suchbild den Installationsort ermittelt hat.

Anmerkung: Die Begriffe "Referenzbild" und "Suchbild" bezeichnen, in welcher Phase das jeweilige Bild aufgenommen worden ist. Technisch können die beiden Bilder übereinstimmen und insbesondere dasselbe Datenformat aufweisen. Sie können auch mit demselben Bildaufnahmegerät erzeugt worden sein.

Die Bildvergleichseinheit kann ein Bestandteil des Suchgeräts sein oder räumlich vom Suchgerät entfernt sein.

In einer Realisierungsform ist die Bildvergleichseinheit ein Bestandteil des Suchgeräts und ist bevorzugt als ein Softwareprogramm realisiert. Die Suchbilder werden also innerhalb des Suchgeräts an die Bildvergleichseinheit übermittelt und brauchen nicht drahtlos übermittelt zu werden. In einer anderen Realisierungsform ist die Bildvergleichseinheit räumlich vom Suchgerät entfernt. Insbesondere ist sie auf dem Zentralrechner oder auf einem weiteren räumlich entfernten Rechner installiert. Diese Realisierungsform erspart die Notwendigkeit, das Suchgerät mit einer ausreichend großen Rechenkapazität und Speicherkapazität versehen zu müssen. Wenigstens zeitweise ist eine bidirektionale Datenverbindung zwischen der Bildvergleichseinheit und dem Suchgerät hergestellt.

In manchen Fällen ist der installierte Sensor wenigstens zeitweise und vollständig oder wenigstens teilweise hinter einem Gegenstand verborgen, und zwar aus jeder Betrachtungsrichtung oder mindestens aus einigen Betrachtungsrichtungen. Beispielsweise befindet der Sensor sich in einem umschlossenen Raum oder unter einem vorspringenden Gebäudeteil. Der umschlossene Raum ist beispielsweise durch eine Tür zugänglich. Um auch in dieser Situation den Servicetechniker rasch zum Sensor zu führen, werden in der Installationsphase bevorzugt ein erstes und ein zweites Referenzbild erzeugt. Das erste Referenzbild zeigt den Sensor am Installationsort. Das zweite Referenzbild zeigt einen Gegenstand, der sich zwischen dem Bildaufnahmegerät des Installationsgeräts und dem Sensor befindet, beispielsweise eine verschlossene Tür zu dem umschlossenen Raum oder einen Behälter, in dem sich der Sensor befindet. Möglich ist, dass mehrere erste Referenzbilder und / oder mehrere zweite Referenzbilder erzeugt werden.

Weiter oben wurde eine Ausgestaltung beschrieben, bei der das Suchgerät das oder jedes Referenzbild als Bestandteil der Installationsort-Information ausgibt. Diese Ausgestaltung lässt sich mit der gerade beschriebenen Ausgestaltung kombinieren, bei der mindestens zwei Referenzbilder erzeugt werden, wobei das erste Referenzbild den Sensor und das zweite Referenzbild den Gegenstand vor dem Sensor zeigt. Das Suchgerät gibt dann diese beiden Referenzbilder aus.

Die optionale Bildvergleichseinheit vergleicht das oder mindestens ein Suchbild mit beiden Referenzbildern. In dem gerade beschriebenen Fall kann das Suchbild die geschlossene oder die geöffnete Tür zeigen, wobei im Suchbild bei geöffneter Tür häufig der Sensor sichtbar ist und bei geschlossener Tür nicht.

Erfindungsgemäß wird die Installationsort-Information unter Verwendung mindestens eines Referenzbilds eines aufzufindenden Sensors ermittelt. In einer gerade beschriebenen Ausgestaltung vergleicht die Bildvergleichseinheit Suchbilder mit Referenzbildern. In einer anderen Ausgestaltung gibt das Suchgerät mindestens ein Referenzbild aus. Beide Ausgestaltungen erfordern, dass die oder wenigstens einige Referenzbilder geeignet sind. Genauer gesagt: Diese Referenzbilder müssen dazu geeignet sein, dass der Vergleich der Suchbilder mit den Referenzbildern eine Information über den Installationsort liefert, wobei diese Information dafür geeignet ist, dass der Servicetechniker den Installationsort auffindet. In der Regel setzt dies voraus, dass mindestens ein Referenzbild den aufzufindenden Sensor und keinen weiteren Sensor zeigt, zumindest keinen weiteren gleichartigen oder gleich aussehenden Sensor. Falls Referenzbilder als Bestandteil der Installationsort-Information ausgegeben werden, so sollte mindestens ein Referenzbild den aufzufindenden Sensor und idealerweise keinen weiteren Sensor zeigen.

Aus diesem Grund umfasst die Betriebs-Anordnung in einer Ausgestaltung zusätzlich zur oder anstelle der Bildvergleichseinheit eine signalverarbeitende Bildauswerteeinheit. Diese Bildauswerteeinheit wird in der Installationsphase eingesetzt. Die Bildauswerteeinheit kann ein Bestandteil des Installationsgeräts oder räumlich vom Installationsgerät entfernt sein, insbesondere ein Bestandteil des Zentralrechners oder eines weiteren Rechners sein. Das oder jedes Referenzbild wird an die Bildauswerteeinheit übermittelt. Die Bildauswerteeinheit und die Bildvergleichseinheit können durch unterschiedliche Softwareprogramme auf demselben Gerät realisiert sein.

Die Ausgestaltung mit der Bildauswerteeinheit umfasst die folgenden Schritte:
- Die Bildauswerteeinheit durchsucht jedes Referenzbild vom Installationsort des Sensors nach einem Abbild eines Sensors der Sensor-Anordnung. Die Bildauswerteeinheit stellt nicht notwendigerweise fest, welcher Sensor im Referenzbild gezeigt wird. Bevorzugt unterscheidet sie ein Abbild eines Sensors der Sensor-Anordnung von Abbildern anderer Gegenstände und zählt, wie viele Sensoren im Referenzbild gezeigt werden.
- In einer Ausgestaltung wird ein rechnerauswertbar Katalog mit Abbildern von Sensoren vorgegeben. Die Bildauswerteeinheit benutzt diesen Katalog, um in den Referenzbilder nach Abbildern von Sensoren zu suchen. In einer Ausgestaltung verwendet die Bildauswerteeinheit vorgegebene Konturen von Sensoren der Sensor-Anordnung.
- Folgendes Ergebnis ist ein Indiz dafür, dass kein geeignetes Referenzbild vorhanden ist: Jedes Referenzbild für einen Sensor zeigt entweder überhaupt kein Abbild eines Sensors oder zwei Abbilder von zwei räumlich voneinander beabstandeten Sensoren, insbesondere zwei gleichartige oder gleich aussehende Sensoren. Falls die Bildauswerteeinheit dieses Ergebnis ermittelt hat, so generiert sie eine entsprechende Nachricht.
- Das Installationsgerät gibt diese Nachricht in mindestens einer von einem Menschen wahrnehmbaren Form aus. Bevorzugt gibt das Installationsgerät zusätzlich jedes Referenzbild aus.

Diese Ausgestaltung erspart die Notwendigkeit, dass der Installationstechniker selbst überprüfen muss, ob mindestens ein geeignetes Referenzbild vorhanden ist. Ermöglicht wird, dass der Installationstechniker bereits am Installationsort darüber informiert wird, dass bislang kein geeignetes Referenzbild vorhanden ist. Als Reaktion hierauf kann der Installationstechniker bereits am Installationsort mindestens ein weiteres Referenzbild anfertigen. Beispielsweise fertigt der Installationstechniker weitere Referenzbilder mit einem geringeren Abstand zum Sensor und / oder aus einer anderen Blickrichtung an.

Die Erfindung erspart die Notwendigkeit, einen Geopositionssensor einzusetzen, um den Servicetechniker zu einem Installationsort zu führen. In einer Ausgestaltung umfassen hingegen sowohl das Installationsgerät als auch das Suchgerät jeweils einen Geopositionssensor. Jeder Geopositionssensor vermag seine eigene aktuelle Geoposition zu messen. Gemäß dieser Ausgestaltung werden in der Installationsphase für mindestens einen, bevorzugt für jeden, Sensor der Sensor-Anordnung zusätzlich folgende Schritte durchgeführt:
- Der Geopositionssensor des Installationsgeräts misst die Geoposition des Installationsorts, an dem der Sensor installiert wird oder ist. Genauer gesagt: Der Geopositionssensor misst seine jeweilige eigene Geoposition, und diese stimmt häufig ausreichend genau mit der Geoposition des Sensors der Sensor-Anordnung überein. Alternativ umfasst der Sensor selbst einen Geopositionssensor, und dieser Geopositionssensor misst die Geoposition des Installationsorts.
- Die gemessene Geoposition des Installationsorts wird vom Installationsgerät an den Zentralrechner übermittelt, bevorzugt zusammen mit den Referenzbildern und / oder mit einer eindeutigen Kennung des Sensors und / oder mit einem Zeitstempel. Der Zeitstempel gibt den Zeitpunkt an, an dem der Sensor am Installationsort installiert worden ist.
- Der Zentralrechner bewirkt folgendes: Der Datensatz, der für den Sensor angelegt oder ergänzt wird, umfasst zusätzlich die übermittelte Geoposition des Installationsorts - genauer gesagt: eine Kennzeichnung dieser Geoposition. Falls der Datensatz bereits angelegt ist, wird er um die übermittelte Geoposition ergänzt.

In der Suchphase wird für den oder jeden aufzufindenden Sensor folgender Schritt durchgeführt:
- Der Geopositionssensor des Suchgeräts misst mindestens einmal seine eigene Geoposition, also die Geoposition des Suchgeräts. Bevorzugt misst der Geopositionssensor des Suchgeräts in der Suchphase mehrmals die jeweilige eigene Geoposition, und das Suchgerät stellt fest, wie sich der Abstand zwischen der aktuellen gemessenen Geopositionen und der Geoposition des Installationsorts verändert.

Möglich ist, die Ausgestaltung, bei der das Suchgerät einen Geopositionssensor umfasst, mit der Ausgestaltung zu kombinieren, dass ein Bildaufnahmegerät des Suchgeräts Suchbilder erzeugt und eine Bildvergleichseinheit die Suchbilder mit den Referenzbildern vergleicht. Bei dieser Kombination wird bevorzugt die jeweilige Messung der Geoposition durchgeführt, während das Bildaufnahmegerät des Suchgeräts das oder ein Suchbild erzeugt.

Erfindungsgemäß wird eine Information über den Installationsort ermittelt, und das Suchgerät gibt diese Installationsort-Information aus. Für die Ermittlung werden die Referenzbilder verwendet, in einer Ausgestaltung das Ergebnis eines Bildvergleichs zwischen den Suchbildern und den Referenzbildern. Gemäß der gerade beschriebenen Ausgestaltung verwendet das Suchgerät zusätzlich folgende Informationen als Bestandteile der Installationsort-Information:
- die abgespeicherte Geoposition des Installationsorts eines aufzufindenden Sensors, wobei diese Geoposition in der Installationsphase gemessen, an den Zentralrechner übermittelt und als ein Bestandteil des Datensatzes für den Sensor abgespeichert ist, und
- die oder jedem Geoposition, die der Geopositionssensor des Suchgeräts in der Suchphase gemessen hat.

Gemäß einer bevorzugten Ausgestaltung werden in der Suchphase eine erste Teilphase und eine nachfolgende zweite Teilphase durchgeführt. In der ersten Teilphase wird ein Signal eines Geopositionssensors verwendet, um einen Servicetechniker zum Installationsort eines aufzufindenden Sensors zu führen - oder wenigstens in die Nähe des Installationsorts. Die oben beschriebenen Begrenzungen, die bei der Nutzung eines Geopositionssensors auftreten, können auch in der ersten Teilphase wirksam werden.

Die Ausgestaltung mit den beiden Teilphasen wird bevorzugt mit folgender Ausgestaltung kombiniert: Das Suchgerät vermag eine Kennung eines aufzufinden Sensors und / oder eine Eingabe eines Servicetechnikers, einen aufzufinden Sensor gefunden zu haben, zu erfassen.

In der ersten Teilphase werden bevorzugt keine Referenzbilder und keine Suchbilder verwendet, bevorzugt aber Geopositionen. Möglich ist auch, dass in der ersten Teilphase das oder mindestens ein, bevorzugt jedes Referenzbild vom Installationsort des aufzufindenden Sensors an das Suchgerät übermittelt wird. Das Suchgerät gibt in der Suchphase das oder jedes empfangene Referenzbild visuell aus. Bei dieser alternativen Ausgestaltung umfassen weder das Installationsgerät noch das Suchgerät notwendigerweise einen Geopositionssensor.

In der ersten Teilphase wird die optionale Bildvergleichseinheit des Suchgeräts nicht verwendet, wohl aber in der zweiten Teilphase.

Die zweite Teilphase wird durchgeführt, wenn der Servicetechniker in der ersten Teilphase noch nicht den Sensor aufgefunden hat - genauer gesagt: wenn das Suchgerät weder die oben beschriebene Kennung noch die oben beschriebene Eingabe erfasst hat. In der zweiten Teilphase wird mindestens ein Referenzbild mit mindestens einem Suchbild verglichen, um den Servicetechniker von dem Ort, an dem der Servicetechniker sich am Ende der ersten Teilphase befindet, zum Installationsort zu führen. Diesen Vergleich führt bevorzugt die optionale Bildvergleichseinheit durch. Der Geopositionssensor wird nicht notwendigerweise in der zweiten Teilphase verwendet. Möglich ist aber, auch in der zweiten Phase ein Signal eines Geopositionssensors zu verwenden, und zwar zusätzlich zu dem oder jedem Referenzbild und zu den Suchbildern.

Bei dieser Ausgestaltung werden das Signal des Geopositionssensors und optional die Referenzbilder verwendet, um in der ersten Teilphase den Servicetechniker in die Nähe des Installationsorts zu führen. Mindestens dann, wenn der Servicetechniker den Installationsort nicht bereits in der ersten Teilphase findet, vergleicht die Bildvergleichseinheit zusätzlich die Suchbilder und das oder mindestens ein Referenzbild automatisch miteinander, um den Servicetechniker zum Installationsort zu führen. Diese Kombination vergrößert in vielen Fällen die Zuverlässigkeit, dass der Servicetechniker rasch den Installationsort findet, verglichen mit einer Ausgestaltung, bei der nur ein Signal eines Geopositionssensors oder nur Referenzbilder verwendet werden. In vielen Fällen reduziert diese Kombination außerdem die Anzahl von Suchbildern, die angefertigt und mit dem Referenzbild oder den Referenzbildern verglichen werden müssen, bis der Servicetechniker den Installationsort gefunden hat, verglichen mit einer Ausgestaltung, bei der ausschließlich Referenzbilder und Suchbilder verwendet werden. Diese Ausgestaltung spart also Bandbreite ein, weil weniger Bilder übermittelt werden müssen.

Bei der gerade beschriebenen Ausgestaltung mit der ersten Teilphase und der zweiten Teilphase wird die zweite Teilphase dann durchgeführt, wenn die erste Teilphase durchgeführt worden ist und das Suchgerät keine Eingabe erfasst hat, dass das Sensor nunmehr gefunden ist, sondern überhaupt keine entsprechende Eingabe oder explizit eine Eingabe, dass die Suche fortgesetzt werden soll.

Gemäß der weiter oben beschriebenen Ausgestaltung wird das Ergebnis des Bildvergleichs an das Suchgerät übermittelt. Das Suchgerät gibt eine Information über den Installationsort in mindestens einer von einem Menschen wahrnehmbaren Form aus. Unterschiedliche Ausgestaltungen sind möglich, wie das Suchgerät die Installationsort-Information ausgibt. Mindestens zwei dieser Ausgestaltungen lassen sich miteinander kombinieren.

In einer Ausgestaltung sucht die optionale Bildvergleichseinheit in den Suchbildern nach einem Abbild des aufzufindenden Sensors. Für diese Suche verwendet die Bildvergleichseinheit mindestens ein Referenzbild vom Installationsort des aufzufindenden Sensors. Bevorzugt verwendet die Bildvergleichseinheit für die Suche ein Referenzbild, welches genau einen Sensor zeigt. In einer Realisierungsform ist in diesem Referenzbild eine eindeutige Kennung oder ein sonstiges visuell erfassbare Merkmal des Sensors sichtbar.

Falls die Bildvergleichseinheit in einem Suchbild den aufzufindenden Sensor aufgefunden hat, so führt das Suchgerät folgenden Schritt durch: Das Suchgerät gibt das Suchbild aus, bevorzugt visuell, wobei dieses Suchbild den Sensor zeigt. In diesem Suchbild zeigt bevorzugt eine automatisch oder manuell erzeugte Markierung einen Bildbereich des Suchbilds, wobei der Bildbereich den aufzufindenden Sensor zeigt. Besonders bevorzugt hat die Bildvergleichseinheit zuvor eine Kennzeichnung dieses Bildbereichs generiert, und diese Kennzeichnung wurde ebenfalls an das Suchgerät übermittelt. Möglich ist auch, dass das Suchgerät selbst diese Markierung erzeugt. Die Ausgestaltung mit dem Suchbild und der Markierung erleichtert es, den Sensor am Installationsort zu finden.

Gemäß dieser Ausgestaltung wird ein Suchbild ausgegeben, welches den aufzufindenden Sensor zeigt. Diese Ausgestaltung ermöglicht es in vielen Fällen, den Sensor auch dann zu finden, wenn die Umgebung des Sensors seit der Installationsphase verändert worden ist. Das Suchbild wurde nämlich in der Suchphase angefertigt, nicht in der vorhergehenden Installationsphase, und ist daher aktuell.

In einer Ausgestaltung werden in der Suchphase bei der Suche nach einem Sensor mehrere Suchbilder angefertigt. Jedes Suchbild wird mit dem oder mindestens einem, bevorzugt mit jedem Referenzbild verglichen. Für jedes Suchbild wird jeweils ein Übereinstimmungs-Maß mit dem zum Vergleich herangezogenen Referenzbild berechnet. Dieses Übereinstimmungs-Maß ist ein Maß dafür, wie gut das Suchbild mit dem Referenzbild übereinstimmt. Die Vergleiche und die Berechnungen führt die Bildvergleichseinheit durch. In einer Ausgestaltung verwendet die Bildvergleichseinheit zum Bildvergleich nur solche Bildbereiche, die jeweils einen Sensor zeigen. Bevorzugt verwendet die Bildvergleichseinheit nur Bilder, welche jeweils einen Sensor zeigen, also insbesondere kein Referenzbild, welches einen Gegenstand zwischen dem Sensor und der Kamera zeigt. Falls im Datensatz für einen Sensor mehrere Referenzbilder hinterlegt sind, werden bevorzugt für jedes Suchbild jeweils mehrere Übereinstimmungs-Maße berechnet. Als das Übereinstimmungs-Maß des Suchbilds wird bevorzugt das größte dieser Übereinstimmungs-Maße verwendet.

Mindestens ein Suchbild wird ausgewählt. Das oder jedes ausgewählte Suchbild wird als Bestandteil der Installationsort-Information auf dem Suchgerät ausgegeben. Beispielsweise wird das Suchbild mit dem größten Übereinstimmungs-Maß ausgewählt. Oder jedes Suchbild wird ausgewählt, dessen Übereinstimmungs-Maß größer ist als eine vorgegebene untere Übereinstimmungs-Schranke. In einer Ausgestaltung wählt die Bildvergleichseinheit die Suchbilder aus. Eine Kennzeichnung der ausgewählten Suchbilder wird an das Suchgerät übermittelt. Möglich ist auch, dass die Übereinstimmungs-Maße an das Suchgerät übermittelt werden und das Suchgerät selbst Suchbilder auswählt, und zwar abhängig von den Übereinstimmungs-Maßen.

Möglich ist, dass kein Suchbild ausgewählt wird, beispielsweise weil kein Suchbild ein ausreichend großes Übereinstimmungs-Maß aufweist. In diesem Fall gibt bevorzugt das Suchgerät eine Meldung an den Servicetechniker aus, wobei die Meldung die Aufforderung umfasst, mindestens ein weiteres Suchbild anzufertigen.

In vielen Fällen erleichtert das oder jedes ausgegebene Suchbild es einem Servicetechniker, den Sensor aufzufinden. Dank der Übereinstimmungs-Maße ist die Gefahr geringer, dass ein ungeeignetes oder falsches Suchbild ausgegeben wird.

Erfindungsgemäß gibt das Suchgerät eine Installationsort-Information aus. In einer Ausgestaltung umfasst diese Installationsort-Information eine Kennzeichnung eines Wegs, der zum aufzufindenden Sensor führt. Dieser Weg beginnt bevorzugt an einem Ort, an dem mindestens ein Suchbild erzeugt worden ist oder zu dem der Servicetechniker aufgrund eines Signals des Geopositionssensors des Suchgeräts und / oder aufgrund eines ausgegebenen Referenzbilds geführt worden ist, insbesondere in der oben erwähnten ersten Teilphase. Diesen Weg ermittelt das Suchgerät. Gemäß dieser Ausgestaltung misst das Suchgerät bei der Erzeugung eines Suchbilds die Blickrichtung, in die dieses Suchbild angefertigt worden ist. Diese Blickrichtung bezieht sich beispielsweise auf ein globales Koordinatensystem. Um den Weg zum Installationsort zu ermitteln, verwendet das Suchgerät
- das Ergebnis des Bildvergleichs und
- die ermittelten Blickrichtungen der Suchbilder,
optional zusätzlich
- die Geoposition des Installationsorts, die in der Installationsphase gemessen worden ist, und
- die jeweilige Geoposition, an dem ein Suchbild erzeugt worden ist, die also in der Suchphase gemessen worden ist.

Die Ausgestaltung, dass das Suchgerät einen Weg zum Installationsort ermittelt und ausgibt, erleichtert es in vielen Fällen, den Installationsort aufzufinden, und zwar auch dann, wenn dieser Installationsort relativ verborgen ist.

In einer Ausgestaltung ist mindestens ein Sensor der Sensor-Anordnung in einem Gebäude oder Fahrzeug mit mehreren Stockwerken installiert. Damit ein Servicetechniker diesen Sensor findet, muss er den Sensor im richtigen Stockwerk finden. In einer Ausgestaltung zeigt mindestens ein Referenzbild, welches in der Installationsphase von diesem Sensor erzeugt worden ist, eine Kennzeichnung des Stockwerks, in dem der Sensor installiert ist. Auch dieses Referenzbild gehört zum Datensatz für den Sensor. Dieses Referenzbild zeigt nicht notwendigerweise den Sensor selbst. In der Suchphase erleichtert dieses Referenzbild es dem Servicetechniker, das richtige Stockwerk zu finden.

In einer Ausgestaltung umfasst mindestens ein Sensor der Sensor-Anordnung eine Kommunikationseinheit. Auch das Suchgerät umfasst eine Kommunikationseinheit. Dank der beiden Kommunikationseinheiten vermag der Sensor ein Signal an das Suchgerät zu übermitteln. Beispielsweise sendet das Suchgerät eine Anfrage aus, welche eine Kennung eines aufzufindenden Sensors umfasst, und der aufzufindende Sensor sendet eine Antwort. Möglich ist auch, dass der Sensor regelmäßig Nachrichten verschickt, beispielsweise um einem räumlich entfernten Empfänger mitzuteilen, dass der Sensor noch intakt und aktiv ist.

In einer Ausgestaltung umfasst die Suchphase für mindestens einen aufzufindenden Sensor die folgenden zusätzlichen Schritte:
Mindestens einmal wird der aktuelle Abstand zwischen dem aufzufindenden Sensor und dem Suchgerät gemessen. Bevorzugt wird dieser Abstand wiederholt gemessen. In einer Realisierungsform wird die gerade beschriebene Datenverbindung zwischen dem Sensor und dem Suchgerät verwendet, um den Abstand zu messen. Der Abstand lässt sich natürlich nur dann mithilfe der Datenverbindung messen, wenn tatsächlich eine Datenverbindung hergestellt ist. Möglich ist, dass der Abstand zusätzlich oder alternativ mithilfe der oben beschriebenen Geoposition des Sensors und des Suchgeräts ermittelt werden. Falls der Abstand sowohl mithilfe der Datenverbindung als auch mithilfe der Geoposition ermittelt wird, ist eine Plausibilitätsprüfung möglich, und außerdem wird Redundanz hergestellt.

Das Suchgerät gibt als Bestandteil der Installationsort-Information mindestens eine der folgenden Informationen aus:
- eine Kennzeichnung für den gemessenen Abstand,
- eine Kennzeichnung, ob der gemessene Abstand größer wird, kleiner wird oder gleich bleibt,
- optional mindestens ein Referenzbild des aufzufindenden Sensors,
- optional die Geoposition des Installationsorts und
- optional eine Kennung des aufzufindenden Sensors, wobei diese Kennung vom aufzufindenden Sensor an das Suchgerät übermittelt worden ist.

In vielen Fällen erleichtert es diese Ausgestaltung dem Servicetechniker noch besser, den Sensor zu finden. Der Servicetechniker wird darüber informiert, wie weit er noch vom Sensor entfernt ist und ob er sich in die richtige oder in eine falsche Richtung relativ zum Sensor bewegt.

Erfindungsgemäß vermag der oder jeder Sensor der Sensor-Anordnung jeweils eine physikalische Größe zu messen. Unterschiedliche Ausgestaltungen sind möglich, welche physikalische Größe dies jeweils ist.

In einer Ausgestaltung ist der oder jeder oder mindestens ein Sensor der Sensor-Anordnung ein Gasmessgerät. Als physikalische Größe vermag der Sensor in einer ersten Alternative die aktuelle Konzentration eines Zielgases zu messen, insbesondere die eines brennbaren oder auf andere Weise schädlichen Zielgases oder auch eines für Menschen lebensnotwendigen Zielgases, insbesondere Sauerstoff. In einer zweiten Alternative vermag der Sensor automatisch zu entscheiden, ob die Konzentration des Zielgases kleiner oder größer als eine vorgegebenen Schranke ist. Insbesondere bei brennbaren oder sonstigen schädlichen Zielgasen darf die Konzentration nicht größer als eine vorgegebene obere Schranke sein. Umgekehrt muss die Konzentration von Sauerstoff größer als eine vorgegebene untere Schranke sein. Das Zielgas kann auch ein Narkosemittel in einem Gasgemisch oder ein sonstiger Bestandteil dieses Gasgemischs sein, welches zu einem Patienten gefördert wird, oder Narkosemittel in der Umgebungsluft. Die Narkosemittel-Konzentration im Gasgemisch soll in einem vorgegebenen Bereich liegen, der in der Regel sowohl nach oben als auch nach unten durch jeweils eine Schranke begrenzt ist.

Möglich ist auch, dass der oder mindestens ein Sensor der Sensor-Anordnung eine Umgebungsbedingung zu messen vermag, insbesondere die Umgebungstemperatur, die Umgebungsfeuchte oder den Umgebungsdruck oder die Windrichtung und / oder Windstärke und / oder Lichtstärke. Der oder ein Sensor kann auch dazu ausgestaltet sein, eine Niederschlagsmenge zu messen oder Feuer oder Rauch oder Staub oder Tau oder Nebel zu detektieren. Eine weitere Anwendung ist, dass der Sensor einen Schallpegel oder eine Schallfrequenz oder die Intensität oder den Wellenlängen-Bereich einer elektromagnetischen Strahlung misst. Beispielsweise vermag der Sensor mit Hilfe von Ultraschall ein Leck in einem Rohr oder einer sonstigen Fluidführungseinheit zu detektieren oder auszuschließen, dass ein Leck vorliegt. Oder der Sensor vermag ein Maß für die Lärmbelastung oder Strahlenbelastung an einem bestimmten Ort zu messen. Der Sensor kann auch einen Bewegungsdetektor oder Geräuschdetektor umfassen, wobei der Bewegungsdetektor / Geräuschdetektor eine Bewegung oder ein Geräusch in einem Abtastbereich zu detektieren vermag.

Ein Sensor im Sinne der Ansprüche kann auch eine Kamera sein, die Bilder im sichtbaren Bereich oder auch im Infrarotbereich oder Ultraviolettbereich erzeugt. Ein weiter oben beschriebenen Sensor kann eine solche Kamera umfassen.

Eine mögliche Anwendung ist die folgende: Die Sensor-Anordnung umfasst eine Kamera und ein Gasmessgerät und wird dafür verwendet, um einen Arbeitsort zu überwachen, an dem mindestens ein Arbeiter eine Arbeit durchführt. Die Signale dieser beiden Sensoren werden an eine räumlich entfernte Zentrale übermittelt. Dadurch vermag ein Operator in der Zentrale insbesondere mindestens eines der folgenden unerwünschten Ereignisse zu detektieren: Am Arbeitsort tritt ein gefährliches Zielgas aus. Am Arbeitsort hat sich ein Unfall ereignet. Eine Person am Arbeitsort trägt nicht die vorgeschriebene Schutzausrüstung.

In einer bevorzugten Ausgestaltung umfasst mindestens ein Sensor, bevorzugt jeder Sensor, der Sensor-Anordnung jeweils eine Kommunikationseinheit. Die oder jede Kommunikationseinheit vermag eine Nachricht zu erzeugen und zu veranlassen, dass die Nachricht an einen räumlich entfernten Empfänger übermittelt wird. Diese Nachricht umfasst eine Information über mindestens ein Messergebnis des oder eines Sensors der Sensor-Anordnung und bevorzugt zusätzlich einen Zeitstempel der Messung. Das Messergebnis umfasst einen gemessenen Wert einer physikalischen Größe und/oder eine Aussage, ob der aktuelle Wert der Größe in einen vorgegebenen Wertebereich fällt oder nicht. Diese Nachricht wird per Funkwellen und / oder per Kabel an den räumlich entfernten Empfänger übermittelt. Bevorzugt umfasst der Empfänger eine Ausgabeeinheit, auf der ein von einem Sensor empfangenes Signal in mindestens einer von einem Menschen wahrnehmbaren Form ausgegeben wird. Bevorzugt vermag die Kommunikationseinheit wiederholt eine solche Nachricht zu erzeugen und zu übermitteln.

Bevorzugt umfasst jeder Sensor der Sensor-Anordnung jeweils eine eigene Spannungsversorgungseinheit, aber nicht notwendigerweise eine Ausgabeeinheit, wobei diese Ausgabeeinheit eine Nachricht über eine gemessene Zielgas-Konzentration in einer von einem Menschen wahrnehmbaren Weise ausgibt. Im allgemeinen umfasst der Sensor eine Ausgabeeinheit, die einen Zustand des Sensors ausgibt. Möglich ist auch, dass mindestens ein Sensor sich dauerhaft oder wenigstens zeitweise mit einem stationären Spannungsversorgungsnetz verbinden lässt.

Im Folgenden wird die Erfindung anhand eines Ausführungsbeispiels beschrieben. Hierbei zeigt
- Figur 1: Schritte in der Installationsphase: Drei Gasmessgeräte werden installiert, und von jedem Installationsort wird jeweils mindestens ein Referenzbild angefertigt;
- Figur 2: ein Detail der Ausgestaltung gemäß Figur 1 in einer Draufsicht: Zwei Referenzbilder von demselben Installationsort werden erzeugt;
- Figur 3: ein weiterer Schritt in der Installationsphase: In der zentralen Datenbank wird für die drei Gasmessgeräte jeweils ein Datensatz angelegt, der ein Referenzbild umfasst;
- Figur 4: eine Ausgestaltung der Suchphase: Die Referenzbilder des aufzufinden Gasmessgeräts werden ausgegeben;
- Figur 5: erste Teilphase der Suchphase: Die Geoposition des aufzufindenden Gasmessgeräts wird ermittelt und verwendet;
- Figur 6: zweite Teilphase der Suchphase: Das aufzufindende Gasmessgerät wird in einem Suchbild erkannt;
- Figur 7: zweite Teilphase der Suchphase: Ein Gegenstand, hinter dem sich das Gasmessgerät befindet, wird erkannt;
- Figur 8: zweite Teilphase der Suchphase: Aufgrund der Blickrichtungen beim Erzeugen der Suchbilder wird ein Weg zum Installationsort berechnet;
- Figur 9: zweite Teilphase der Suchphase: Der gemäß Figur 8 berechnete Weg wird auf zwei Weisen angezeigt;
- Figur 10: zweite Teilphase der Suchphase: Auf dem Bildschirm des Suchgeräts werden das Referenzbild und der gemessene Abstand angezeigt.

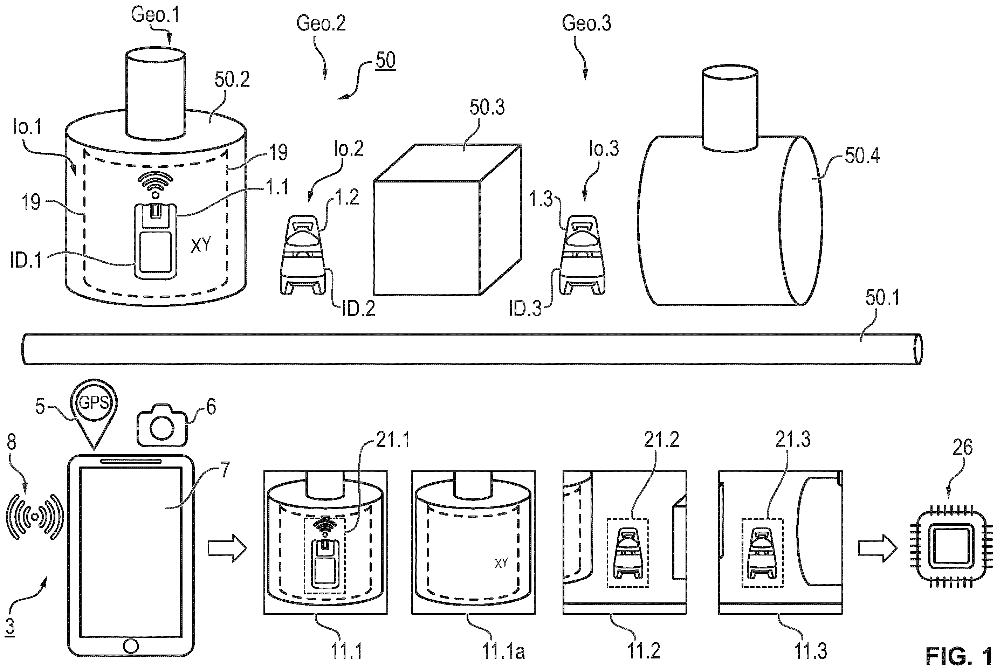

Im Ausführungsbeispiel wird die Erfindung in einer Raffinerie oder einer sonstigen Produktionsanlage 50 eingesetzt. Diese Produktionsanlage 50 umfasst mehrere Bestandteile 50.1, 50.2, 50.3, 50.4, die in Figur 1 schematisch gezeigt werden. Die Sensoren der Sensor-Anordnung sind im Ausführungsbeispiel Gasmessgeräte.

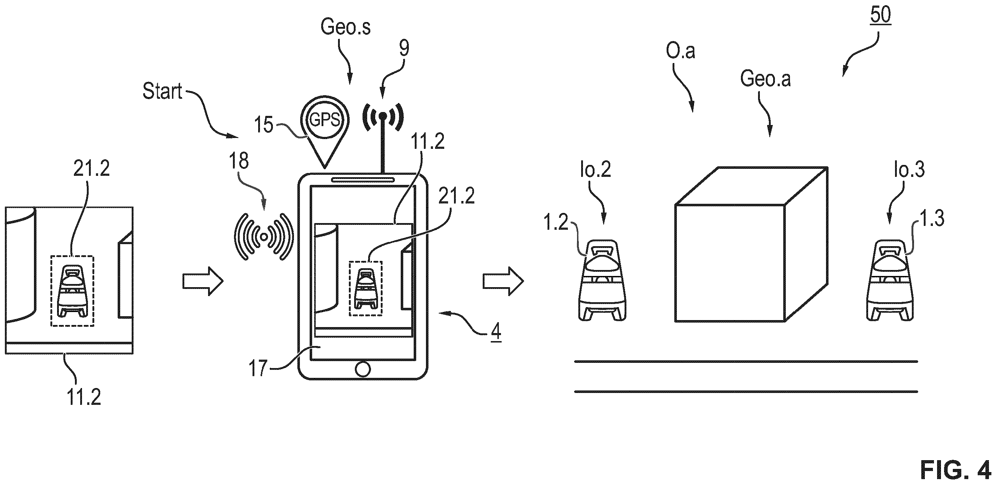

Die Erfindung umfasst eine Installationsphase und eine nachfolgende Suchphase. Figur 1 bis Figur 3 beziehen sich auf die Installationsphase, Figur 4 bis Figur 10 auf die Suchphase. Die Figuren sind nicht notwendigerweise maßstabsgerecht.

In der Installationsphase installiert ein Installationstechniker mehrere stationäre Gasmessgeräte einer Gasmess-Anordnung an verschiedenen Orten in der Produktionsanlage 50. Beispielhaft werden in Figur 1 drei Gasmessgeräte 1.1, 1.2, 1.3 gezeigt, die an drei verschiedenen und räumlich voneinander beanstandeten Installationsorten Io.1, Io.2, Io.3 installiert sind. Der Vorgang, ein Gasmessgerät 1.1, 1.2, 1.3 zu installieren, kann den Schritt umfassen, das Gasmessgerät 1.1, 1.2, 1.3 an einer Wand oder einer Decke zu befestigen und optional das Gasmessgerät 1.1, 1.2, 1.3 mit einem stationären Spannungsversorgungsnetz und / oder einem Datenverbindungsnetz zu verbinden. Möglich ist auch, dass das Gasmessgerät 1.1, 1.2, 1.3 auf einen Boden oder sonstigen Untergrund gestellt wird. In der Regel gehört zu dem Vorgang weiterhin mindestens einer der Schritte, das Gasmessgerät 1.1, 1.2, 1.3 am Installationsort Io.1, Io.2, Io.3 zu überprüfen, zu kalibrieren und in Betrieb zu nehmen.

Jedes Gasmessgerät 1.1, 1.2, 1.3 des Ausführungsbeispiels ist dazu ausgestaltet, in seiner Umgebung jeweils mindestens ein Zielgas zu detektieren und / oder die Konzentration des Zielgases zu messen. Das oder jedes zu detektierende Zielgas ist für einen Menschen schädlich, wenn es in einer zu großen Konzentrationen auftritt, und ist insbesondere ein brennbares und / oder toxisches Gas.

Die Gasmessgeräte 1.1, 1.2, 1.3 verbleiben während des Einsatzes an jeweils einem bestimmten Ort in der Produktionsanlage 50. Möglich ist, dass ein Gasmessgerät 1.1, 1.2, 1.3 an ein stationäres Spannungsversorgungsnetz angeschlossen wird. Möglich ist auch, dass ein Gasmessgerät 1.1, 1.2, 1.3 eine eigene Spannungsversorgungseinheit umfasst.

Im gezeigten Beispiel vermag jedes Gasmessgerät 1.1, 1.2, 1.3 bei einer zu hohen gemessenen Zielgas-Konzentration einen Alarm sowie optional eine gemessene Zielgas-Konzentration an eine räumlich entfernte Zentrale (nicht gezeigt) zu übermitteln, außerdem bevorzugt regelmäßig eine Nachricht, dass das Gasmessgerät 1.1, 1.2, 1.3 noch funktionsfähig ist. Das Gasmessgerät 1.1 ist mit dieser Zentrale drahtlos über Funkwellen verbunden, während die beiden anderen Gasmessgeräte 1.2, 1.3 über eine kabelgebundene Datenverbindung (nicht gezeigt) mit der Zentrale verbunden sind. Diese Realisierungsformen sind nur beispielhaft zu verstehen. In der Zentrale werden Nachrichten von den Gasmessgeräten in mindestens einer von einem Menschen wahrnehmbaren Form ausgegeben.

Jedes Gasmessgerät 1.1, 1.2, 1.3 umfasst im Ausführungsbeispiel jeweils eine eindeutige Kennung ID.1, ID.2, ID.3. In einer Realisierungsform ist diese eindeutige Kennung in einer maschinenlesbaren Form auf eine Oberfläche des Gasmessgeräts 1.1, 1.2, 1.3 aufgebracht, beispielsweise als Abfolge alphanumerischer Zeichen, Barcode oder RFID-Chip. In einer anderen Realisierungsform ist die Kennung ID.1, ID.2, ID.3 als alphanumerische Zeichenfolge dergestalt aufgebracht, dass ein Mensch die Kennung lesen kann.

Der Installationstechniker führt ein mobiles datenverarbeitendes Gerät 3 mit sich, das als das Installationsgerät im Sinne der Ansprüche fungiert. Beispielhaft wird in Figur 1 ein Smartphone 3 gezeigt. Das Installationsgerät 3 umfasst im Ausführungsbeispiel eine Kamera oder ein sonstiges Bildaufnahmegerät 6, einen Geopositionssensor 5, ein Lesegerät (Scanner) 8, eine Ausgabeeinheit / Eingabeeinheit, beispielsweise einen berührungssensitiven Bildschirm (touch screen) 7, sowie eine Recheneinheit (Prozessor) und einen Datenspeicher. Das Bildaufnahmegerät 6 vermag digitale Bilder von seiner Umgebung zu erzeugen. Diese Bilder werden nachfolgend als Referenzbilder bezeichnet. Der Geopositionssensor 5 vermag seine eigene Geoposition zu messen. Das Lesegerät 8 vermag eine maschinenlesbare Kennung ID.1, ID.2, ID.3 auf einem Gasmessgerät 1.1, 1.2, 1.3 zu lesen.

Der Installationstechniker bewirkt, dass das Installationsgerät 3 folgende Schritte durchführt, nachdem der Installationstechniker ein Gasmessgerät 1.1, 1.2, 1.3 an einem bestimmten Ort in der Produktionsanlage 50 installiert und in Betrieb genommen hat:
- Das Lesegerät 8 liest die Kennung ID.1, ID.2, ID.3 des Gasmessgeräts 1.1, 1.2, 1.3. Bevorzugt wird die gelesene Kennung ID.1, ID.2, ID.3 ausgegeben, und der Installationstechniker überprüft, ob die Kennung ID.1, ID.2, ID.3 korrekt gelesen worden ist oder nicht. Oder der Installationstechniker gibt die Kennung ID.1, ID.2, ID.3 in das Installationsgerät 3 ein.
- Das Bildaufnahmegerät 6 erzeugt mindestens ein Referenzbild 11.1, 11.1a, 11.2, 11.3 von dem Installationsort Io.1, Io.2, Io.3, an dem das Gasmessgerät 1.1, 1.2, 1.3 installiert ist.
- Mindestens ein Referenzbild 11.1, 11.2, 11.3 zeigt das Gasmessgerät 1.1, 1.2, 1.3.
- Bevorzugt zeigt mindestens ein weiteres Referenzbild einen Gegenstand, der sich in vielen Fällen wenigstens zeitweise zwischen dem Gasmessgerät 1.1, 1.2, 1.3 und einem Menschen in der Nähe des Gasmessgeräts 1.1, 1.2, 1.3 befindet. Beispielsweise zeigt das Referenzbild eine geschlossene Tür zu einem Raum, in dem sich das Gasmessgerät 1.1, 1.2, 1.3 befindet, oder einen sonstigen Gegenstand, der das Gasmessgerät 1.1, 1.2, 1.3 aus mindestens einer Blickrichtung visuell verdeckt.
- Bevorzugt markieren der Installationstechniker oder auch eine optionale Bildauswerteeinheit 26 in jedem Referenzbild 11.1, 11.2, 11.3 jeweils einen Bereich 21.1, 21.2, 21.3, der das Gasmessgerät 1.1, 1.2, 1.3 zeigt - vorausgesetzt das Referenzbild 11.1, 11.2, 11.3 zeigt das Gasmessgerät 1.1, 1.2, 1.3.
- Der Geopositionssensor 5 misst wenigstens näherungsweise die jeweilige Geoposition Geo.1, Geo.2, Geo.3 des Installationsorts Io.1, Io.2, Io.3, an dem das Gasmessgerät 1.1, 1.2, 1.3 installiert worden ist. Alternativ misst ein Sensor 1.1, 1.2, 1.3 selbst die Geoposition seines eigenen Installationsorts Io.1, Io.2, Io.3, und die gemessene Geoposition wird an das Installationsgerät 3 übermittelt.

Diejenigen Gasmessgeräte, für die in der Installationsphase die gerade beschriebenen Schritte durchgeführt werden, fungieren zusammen als die Sensor-Anordnung im Sinne der Ansprüche.

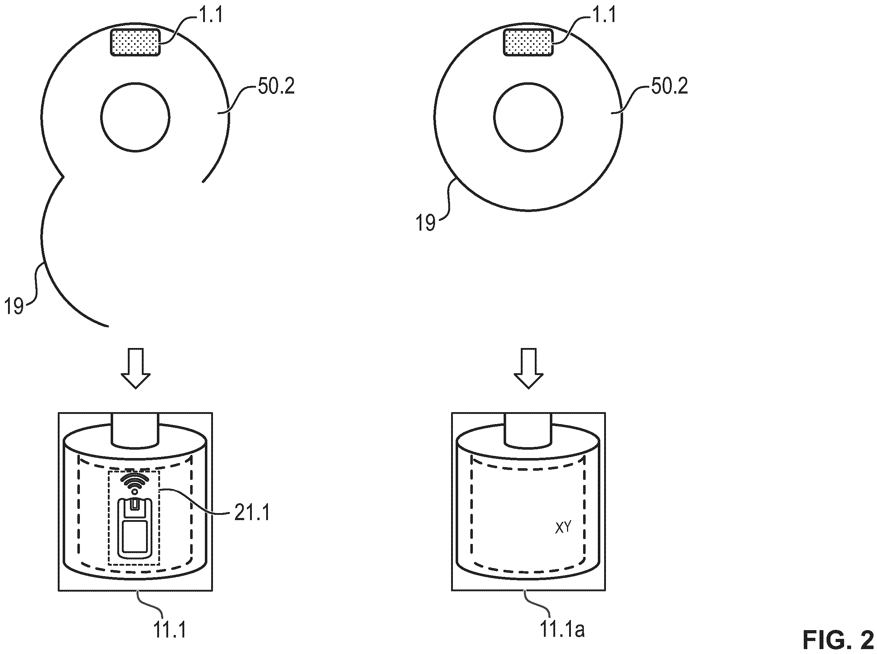

Figur 1 und Figur 2 zeigen schematisch, dass ein Referenzbild 11.1 vom Gasmessgerät 1.1 in einem umschlossenen Raum 50.2 sowie von außen ein weiteres Referenzbild 11.1a vom umschlossenen Raum 50.2 erzeugt wird. Figur 1 ist eine Seitenansicht, Figur 2 eine Draufsicht. Eine Tür 19 vermag den umschlossenen Raum 50.2 wahlweise zu verschließen oder zu öffnen und trägt eine Markierung XY. Figur 2 zeigt links das Referenzbild 11.1 mit dem Gasmessgerät 1.1 bei geöffneter Tür 19. Figur 2 zeigt rechts, wie bei geschlossener Tür 19 das Referenzbild 11.1a angefertigt wird, wobei im Referenzbild 11.1a die geschlossene Tür 19 mit der Markierung XY sichtbar ist, aber nicht das Gasmessgerät 1.1.

In einer Ausgestaltung sucht die Bildauswerteeinheit 26 in den Referenzbildern 11.1, 11.1a, 11.2, 11.3 eines Installationsorts lo.1, Io.2, Io.3 automatisch nach einem Gasmessgerät 1.1, 1.2, 1.3. Die Bildauswerteeinheit 26 prüft, ob folgende unerwünschte Situation eingetreten ist: In jedem Referenzbild 11.1, 11.1a, 11.2, 11.3 von einem Installationsort Io.1, Io.2, Io.3 wird entweder überhaupt kein Abbild eines Gasmessgeräts 1.1, 1.2, 1.3 detektiert, oder die Abbilder von zwei verschiedenen und räumlich voneinander beabstandeten Gasmessgeräten 1.1, 1.2, 1.3 werden detektiert. Die unerwünschte Situation ist eingetreten, wenn die Bildauswerteeinheit 26 dieses Ergebnis mit ausreichend großer Sicherheit erzielt hat. In diesem Fall generiert die Bildauswerteeinheit 26 eine entsprechende Meldung. Diese Meldung wird an das Installationsgerät 3 übermittelt. Das Installationsgerät 3 gibt in mindestens einer von einem Menschen wahrnehmbaren Form eine entsprechende Meldung aus. Beispielsweise wird der Installationstechniker aufgefordert, mindestens ein weiteres Referenzbild vom Installationsort Io.1, Io.2, Io.3 zu erzeugen, wobei die Entfernung zum Gasmessgerät 1.1, 1.2, 1.3 geringer als bei den bisherigen Referenzbildern von diesem Installationsort Io.1, Io.2, Io.3 ist.

Optional läuft auf dem Installationsgerät 3 eine Anwendung ab, die den Installationstechniker dabei unterstützt, die Referenzbilder zu erzeugen. Diese Anwendung kann so ähnlich ausgestaltet sein wie eine Anleitung, die einen Benutzer dabei anleitet, mit einem Smartphone oder einer sonstigen Kamera eine Panoramaaufnahme zu erzeugen. Bei Bedarf unterstützt diese Anwendung den Installationstechniker dabei, weitere Referenzbilder zu erzeugen.

In einer Ausgestaltung ermöglicht es die Anwendung auf dem Installationsgerät dem Installationstechniker, in einem Referenzbild entweder eine Markierung des im Referenzbild dargestellten Gasmessgeräts anzubringen oder die Eingabe zu machen, dass dieses Referenzbild kein Gasmessgerät zeigt.

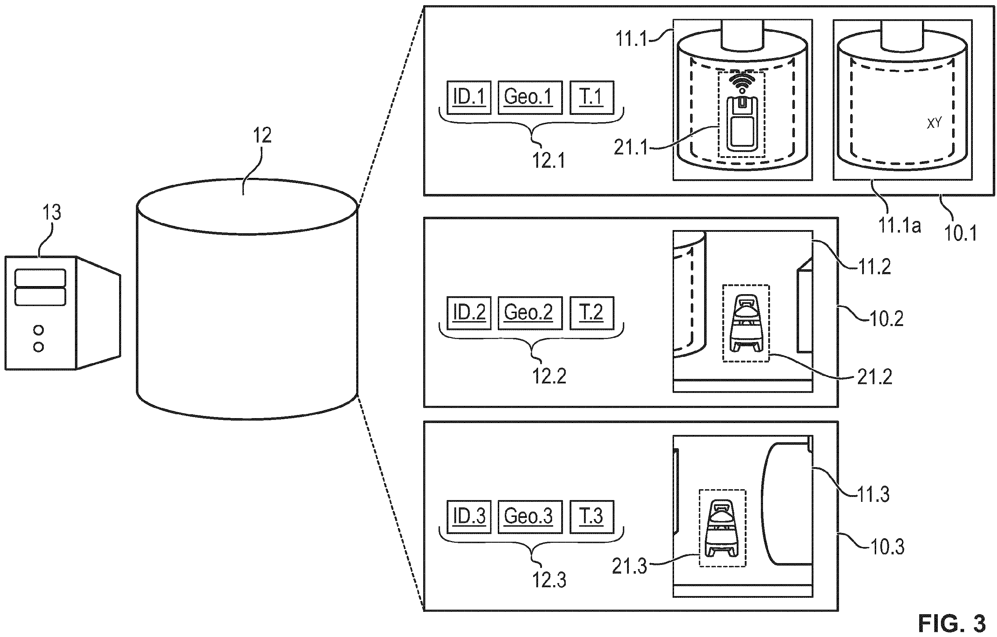

Die Referenzbilder von den Installationsorten Io.1, Io.2, Io.3 und die weiteren Informationen über die Gasmessgeräte 1.1, 1.2, 1.3 werden vom Installationsgerät 3 an eine räumlich entfernte Zentrale und dort an einen Zentralrechner 13 übermittelt. Der Zentralrechner 13 hat Schreibzugriff und Lesezugriff auf eine zentrale Datenbank 12, vgl. Figur 3. Für jedes Gasmessgerät 1.1, 1.2, 1.3, das in der Produktionsanlage 50 installiert ist, ist oder wird in der zentralen Datenbank 12 jeweils ein Datensatz angelegt. Der Zentralrechner 13 bewirkt, dass in der zentralen Datenbank 12 für jedes installierte Gasmessgerät 1.1, 1.2, 1.3 jeweils ein Datensatz 10.1, 10.2, 10.3 angelegt wird. Dieser Datensatz 10.1, 10.2, 10.3 umfasst folgende rechnerauswertbare Informationen über das installierte Gasmessgerät 1.1, 1.2, 1.3:
- die eindeutige Kennung ID.1, ID.2, ID.3 des Gasmessgeräts 1.1, 1.2, 1.3,
- die Geoposition Geo.1, Geo.2, Geo.3 des Installationsorts Io.1, Io.2, Io.3, an dem dieses Gasmessgerät 1.1, 1.2, 1.3 installiert ist,
- der Zeitpunkt T.1, T.2, T.3, an dem das Gasmessgerät 1.1, 1.2, 1.3 installiert oder zum letzten Mal gewartet worden ist,
- das oder mindestens ein Referenzbild 11.1, 11.2, 11.3, welche das installierte Gasmessgerät 1.1, 1.2, 1.3 am Installationsort Io.1, Io.2, Io.3 zeigt, wobei bevorzugt im Referenzbild 11.1, 11.2, 11.3 derjenige Bereich 21.1, 21.2, 21.3 markiert ist, der das Gasmessgerät 1.1, 1.2, 1.3 zeigt, und
- optional ein weiteres Referenzbild 11.1a, welches einen Installationsort Io.1 zeigt, aber nicht notwendigerweise ein Gasmessgerät 1.1, 1.2, 1.3.

Die Kennung ID.1, ID.2, ID.3, die Geoposition Geo.1, Geo.2, Geo.3 und der Zeitpunkt T.1, T.2, T.3 bilden zusammen eine Information 12.1, 12.2, 12.3 im Datensatz 10.1, 10.2, 10.3.

In der gerade genannten Anwendung umfasst die Installationsphase den Schritt, dass der Installationstechniker jedes Gasmessgerät 1.1, 1.2, 1.3 an jeweils einem Installationsort Io.1, Io.2, Io.3 installiert, hierbei die Referenzbilder 11.1, 11.1a, 11.2, 11.3 erzeugt und veranlasst, dass die jeweilige Geoposition Geo.1, Geo.2, Geo.3 und bevorzugt der Zeitpunkt der Installation gemessen werden. Möglich ist auch, dass die Installationsphase durchgeführt wird, nachdem die Gasmessgeräte 1.1, 1.2, 1.3 installiert worden sind, beispielsweise weil die Erfindung auf eine bereits installierte Sensor-Anordnung angewendet werden soll. Der Installationstechniker, der die Schritte der Installationsphase durchführt, installiert also nicht notwendigerweise ein Gasmessgerät 1.1, 1.2, 1.3. Bevorzugt führt der Installationstechniker, der das Installationsgerät 3 mit sich führt und die Referenzbilder 11.1, 11.2, 11.3 erzeugt, zusätzlich Unterlagen über den jeweiligen Installationsort Io.1, Io.2, Io.3 mit sich. Dank der Erfindung werden diese Unterlagen in der Suchphase nicht mehr benötigt.

Von Zeit zu Zeit ist es erforderlich, dass ein Servicetechniker jedes Gasmessgerät 1.1, 1.2, 1.3, das in der Produktionsanlage 50 installiert ist, überprüft. Außerdem kann ein Ereignis erfordern, dass der Servicetechniker ein Gasmessgerät 1.1, 1.2, 1.3 überprüft und bei Bedarf repariert. Beispiele für solche Ereignisse sind:
- Das Gasmessgerät 1.1, 1.2, 1.3 übermittelt an die Zentrale keine Nachricht mehr, funktionsfähig zu sein.
- Das Gasmessgerät 1.1, 1.2, 1.3 hat eine Fehlermeldung an die Zentrale übermittelt, beispielsweise über einen niedrigen Ladezustand der eigenen Spannungsversorgungseinheit oder eine Vergiftung oder einen Ausfall des eigentlichen Sensors.
- Das Gasmessgerät 1.1, 1.2, 1.3 ist dergestalt mit schädlichen Zielgasen belastet, dass es ausgetauscht werden muss.
- Eine vorgesehene Lebensdauer des Gasmessgeräts 1.1, 1.2, 1.3 ist erreicht.

In einer Realisierungsform wählt ein Servicetechniker anhand der eindeutigen Kennung ID.1, ID.2, ID.3 ein Gasmessgerät 1.1, 1.2, 1.3 auf der Produktionsanlage 50 aus. In einer anderen Realisierungsform wird dem Servicetechniker ein Arbeitsauftrag mitgegeben. Dieser Arbeitsauftrag benennt die jeweilige Kennung ID.1, ID.2, ID.3 jedes Gasmessgeräts 1.1, 1.2, 1.3, an dem jetzt eine Arbeit durchzuführen ist, und spezifiziert die jeweils durchzuführende Arbeit. Der Arbeitsauftrag wird bevorzugt vom Zentralrechner 13 unter Verwendung der Datensätze und insbesondere der Zeitstempel T.1, T.2, T.3 erzeugt. Diese Arbeit muss wenigstens teilweise vor Ort durchgeführt werden, kann also nicht ausschließlich aus der Ferne ausgeführt werden. Daher ist es erforderlich, dass der Servicetechniker das oder jedes Gasmessgerät, an dem aktuell Arbeiten auszuführen sind, auf der Produktionsanlage 50 auffindet. Ein solches Gasmessgerät wird im Folgenden als "aufzufindendes Gasmessgerät" bezeichnet.

Im Ausführungsbeispiel sind an den beiden Gasmessgeräten 1.1 und 1.2 Arbeiten auszuführen, und daher sind diese beiden Gasmessgeräte 1.1, 1.2 in der Suchphase aufzufinden. Figur 4 bis Figur 10 veranschaulichen beispielhaft, wie der Servicetechniker zu dem Installationsort Io.1 oder Io.2 geführt wird, an dem das Gasmessgerät 1.1 bzw. 1.2 installiert ist. Der Servicetechniker führt ein mobiles datenverarbeitendes Gerät, beispielsweise ebenfalls ein Smartphone 4, mit sich. Dieses mobile Gerät 4 fungiert als das Suchgerät im Sinne der Ansprüche. Möglich ist, dass dasselbe Gerät sowohl als das Installationsgerät 3 auch als das Suchgerät 4 fungiert. Das Suchgerät 4 umfasst
- einen Geopositionssensor 15,
- ein Bildaufnahmegerät 16,
- einen Bildschirm 17, der bevorzugt berührungssensitiv ist ("touch screen"),
- optional eine Kommunikationseinheit mit einer Antenne 9 und
- optional ein RFID-Lesegerät 18.

An das Suchgerät 4 werden die Informationen 12.1, 12.2 sowie jedes Referenzbild 11.1, 11.1a, 11.2 aus dem Datensatz 10.1, 10.2 für das aufzufindende Gasmessgerät 1.1, 1.2 übermittelt. Beispielsweise wird wenigstens zeitweise eine drahtlose Datenverbindung zwischen der zentralen Datenbank 12 und dem Suchgerät 4 hergestellt, wofür die Kommunikationseinheit mit der Antenne 9 verwendet wird. Die übermittelten Informationen 12.1, 12.2 umfassen im Ausführungsbeispiel die Geoposition Geo.1, Geo.2, an der das Gasmessgerät 1.1, 1.2 installiert ist, sowie in einer Ausgestaltung die Referenzbilder 11.1, 11.1a, 11.2.

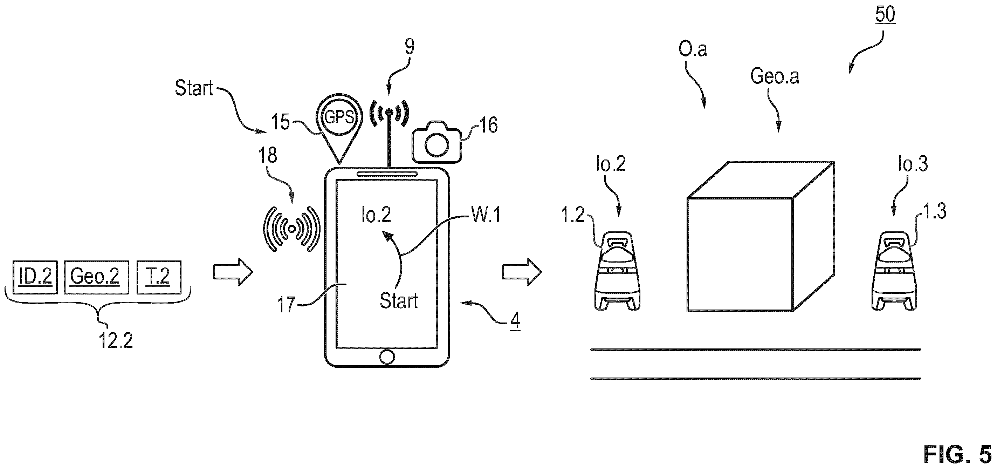

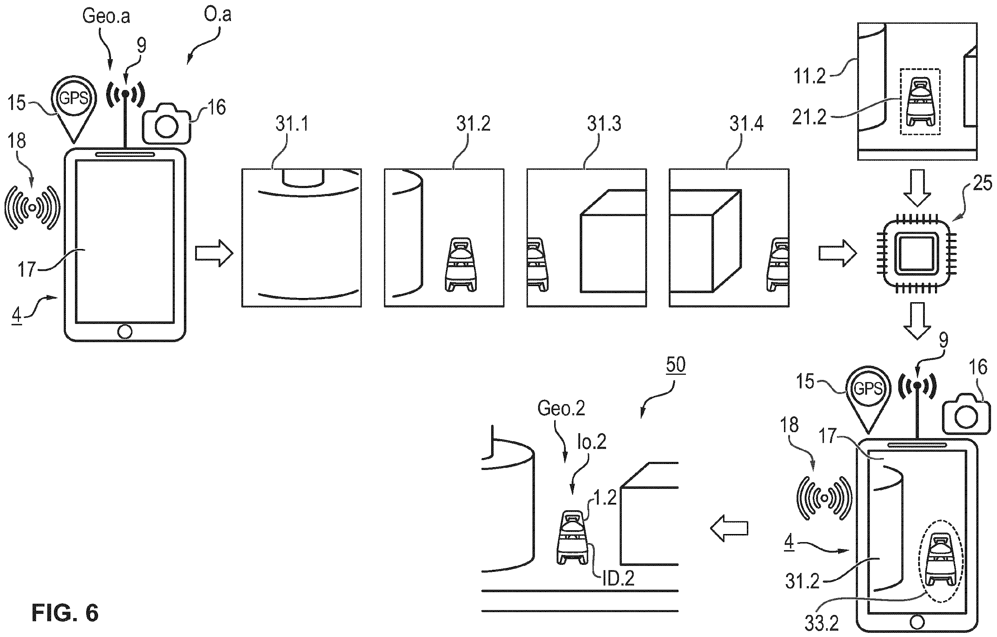

Die Suchphase umfasst im Ausführungsbeispiel eine erste Teilphase, die in Figur 4 und Figur 5 veranschaulicht wird, und eine zweite Teilphase, die in Figur 6 bis Figur 10 veranschaulicht wird.

In der ersten Teilphase führt das Suchgerät 4 den Servicetechniker von einem Ausgangspunkt Start mit der Geoposition Geo.s zum Installationsort Io.2, wenigstens aber zu einem Ort in der Nähe des Installationsorts Io.2. Hierfür verwendet das Suchgerät 4 die übermittelte Geoposition Geo.2 sowie seine eigene aktuelle Geoposition, welche wiederholt mit dem Geopositionssensor 15 gemessen wird. Das Suchgerät 4 berechnet einen Weg W.1 von der Ausgangsposition Start zum Installationsort Io.2 und zeigt eine Information über den berechneten Weg W.1 auf dem Bildschirm 17 an. Hierfür verwendet das Suchgerät 4 die Geoposition Geo.s des Ausgangspunkts Start und die Geoposition Geo.2 des Installationsorts Io.2. Bevorzugt wird das Referenzbild 11.2 aus dem Datensatz 10.2 auf dem Bildschirm 17 des Suchgeräts 4 angezeigt. Die Markierung 21.1 zeigt, wo im Referenzbild 11.2 das Gasmessgerät 1.2 gezeigt wird.

Möglich ist, dass die erste Teilphase bereits ausreicht, um den Servicetechniker zum Installationsort Io.2 zu führen. Jedoch kann es vorkommen, dass die Schritte der ersten Teilphase allein nicht ausreichen, damit der Servicetechniker das Gasmessgerät 1.2 am Installationsort Io.2 findet. Einige mögliche Gründe hierfür sind:
- Beim Installieren des Gasmessgeräts 1.2 wurde die Geoposition Geo.2 des Installationsorts Io.2 nicht exakt genug gemessen.
- Das Suchgerät 4 misst seine eigene aktuelle Geoposition nur näherungsweise, also mit einem Fehler behaftet.
- Das Gasmessgerät 1.2 lässt sich auf allein aufgrund der Geoposition Geo.2 relativ schwierig am Installationsort Io.2 finden, insbesondere weil es am Installationsort verdeckt wird oder in einem Gebäude oder Fahrzeug mit mehreren Stockwerken montiert ist.

In Figur 4 und Figur 5 wird beispielhaft gezeigt, dass der Servicetechniker nicht zum Installationsort Io.2 geführt wird, sondern zu einem Ort O.a, der in der Nähe des gesuchten Installationsorts Io.2 liegt und die Geoposition Geo.a aufweist. Beispielsweise als Reaktion auf eine entsprechende Benutzereingabe wird eine zweite Teilphase durchgeführt. Durch die zweite Teilphase wird der Servicetechniker vom Ort O.a zum Installationsort Io.2 geführt. Diese zweite Teilphase veranschaulicht Figur 6 bis Figur 10. Die zweite Teilphase umfasst die nachfolgend beschriebenen Vorgänge.

Bei der Ausgestaltung gemäß Figur 4 umfasst das Suchgerät 4 nicht notwendigerweise ein Bildaufnahmegerät 16. Eine Bildvergleichseinheit 25 wird ebenfalls nicht benötigt. Das Referenzbild 11.2 vom Installationsort Io.2 wird in der zentralen Datenbank 12 als Bestandteil des Datensatzes 10.2 ermittelt und an das Suchgerät 4 übermittelt. Das Suchgerät 4 zeigt auf seinem Bildschirm 17 das übermittelte Referenzbild 11 an.

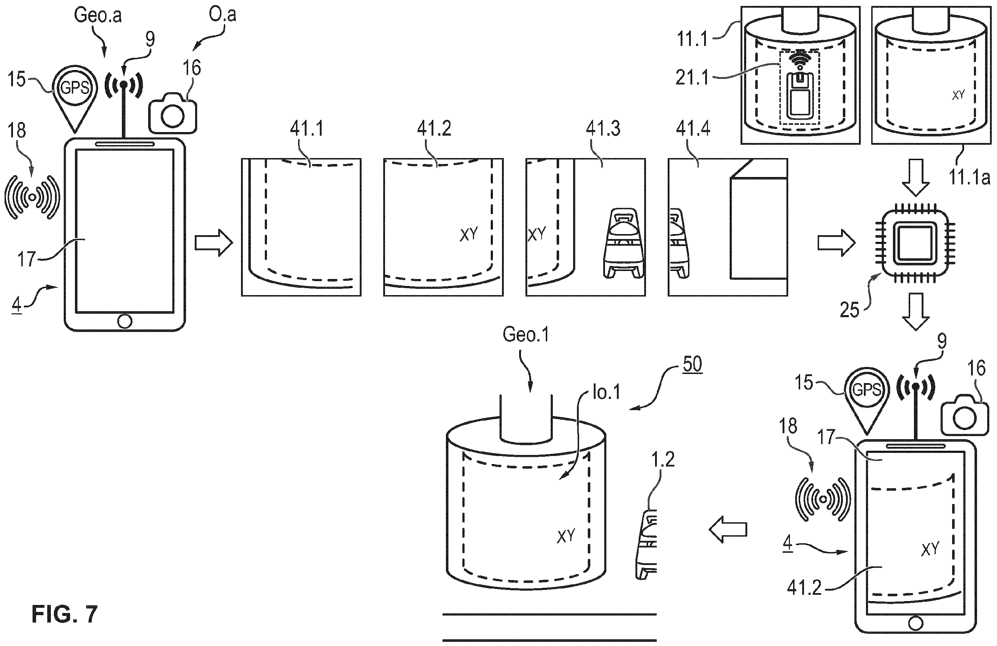

Bei der Ausgestaltung gemäß Figur 5 bis Figur 10 werden ein Bildaufnahmegerät 16 des Suchgeräts 4 sowie eine Bildvergleichseinheit 25 verwendet. Der Servicetechniker veranlasst, dass das Bildaufnahmegerät 16 des Suchgeräts 4 mehrere Bilder von der Umgebung des Orts O.a anfertigt. Diese Bilder werden als Suchbilder bezeichnet. Beispielhaft werden in Figur 6 vier Suchbilder 31.1, 31.2, 31.3, 31.4 und in Figur 7 vier Suchbilder 41.1, 41.2, 41.3, 41.4 gezeigt. Beispielsweise dreht der Servicetechniker sich am Ort O.a einmal um die eigene Achse, und das Bildaufnahmegerät 16 erzeugt eine Abfolge von Suchbildern.

Optional läuft auf dem Suchgerät 4 eine Anwendung ab, die den Servicetechniker dabei unterstützt, die Suchbilder 31.1, 31.2, 31.3, 31.4, 41.1, 41.2, 41.3, 41.4 zu erzeugen. Diese Anwendung kann so ähnlich ausgestaltet sein wie eine Anleitung, die einen Benutzer dabei anleitet, mit der Kamera eines Smartphones oder einer sonstigen Kamera eine Panoramaaufnahme zu erzeugen.

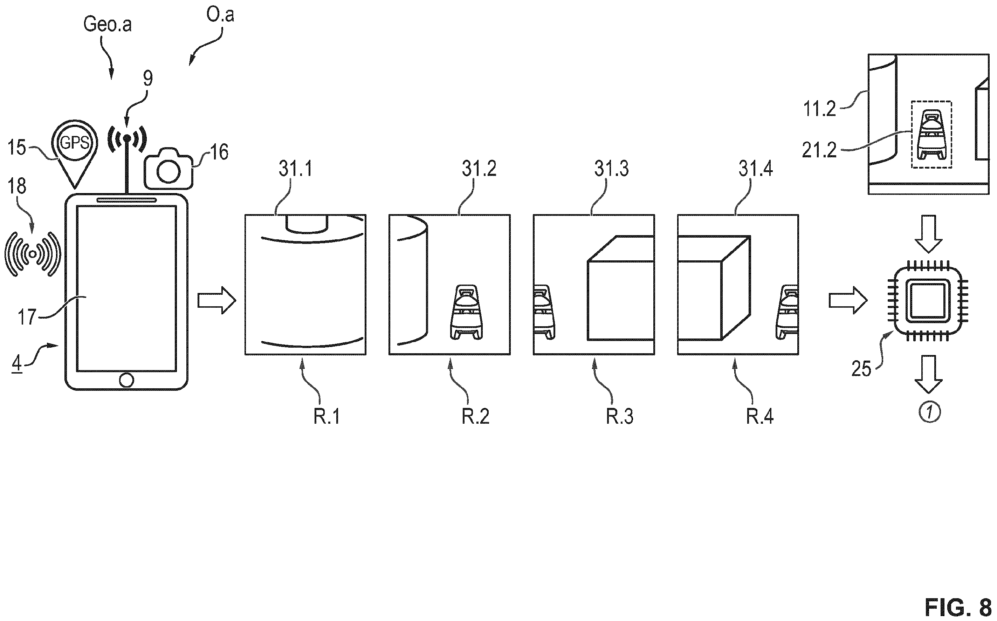

Der Geopositionssensor 15 misst die Geoposition Geo.a des Orts O.a, an dem die Suchbilder 31.1, 31.2, 31.3, 31.4 angefertigt werden. Bevorzugt misst das Suchgerät 4 die jeweilige Blickrichtung R.1, R.2, R.3, R.4, in welche das Bildaufnahmegerät 16 ein Suchbild 31.1, 31.2, 31.3, 31.4 von der Umgebung angefertigt hat, vgl. Figur 8.

Eine Bildvergleichseinheit 25 vergleicht die Suchbilder 31.1, 31.2, 31.3, 31.4, 41.1, 41.2, 41.3, 41.4, welche das Bildaufnahmegerät 16 am Ort O.a angefertigt hat, mit dem oder mindestens einem, bevorzugt jedem Referenzbild 11.1, 11.1a, 11.2 aus dem Datensatz 10.1, 10.2.

In einer Realisierungsform ist die Bildvergleichseinheit 25 ein Teil des Suchgeräts 4. Diese Ausgestaltung erspart eine Datenübermittlung vom Suchgerät 4 an einen anderen Rechner. In einer anderen Realisierungsform gehört die Bildauswerteeinheit 25 zum Zentralrechner 13 oder zu einem sonstigen räumlich entfernten Rechner. Die Suchbilder 31.1, 31.2, 31.3, 31.4, 41.1, 41.2, 41.3, 41.4 werden vom Suchgerät 4 an den Zentralrechner 13 oder an den sonstigen Rechner übermittelt, und dort vergleicht die Bildvergleichseinheit 25 die Suchbilder 31.1, 31.2, 31.3, 31.4, 41.1, 41.2, 41.3, 41.4 mit dem Referenzbild 11.1, 11.1a, 11.2. Das Ergebnis des Bildvergleichs wird zurück an das Suchgerät 4 übermittelt. Die Ausgestaltung, dass die Bildvergleichseinheit 25 räumlich entfernt vom Suchgerät 4 angeordnet ist, erspart die Notwendigkeit, das Suchgerät 4 selbst mit einem ausreichend leistungsfähigen Prozessor versehen zu müssen.

In einer Ausgestaltung sucht die Bildvergleichseinheit 25 in einem Referenzbild 11.1, 11.1a, 11.2 das Abbild vom aufzufindenden Gasmessgerät 1.1, 1.2. Hierfür verwendet die Bildvergleichseinheit 25 bevorzugt die Markierung 21.2. Die Bildvergleichseinheit 25 sucht in den Suchbildern 31.1, 31.2, 31.3, 31.4, 41.1, 41.2, 41.3, 41.4 nach einem Bereich, der ein solches Gasmessgerät zeigt, und hebt in einem solchen Suchbild diesen Bereich hervor.

Im Beispiel von Figur 6 ist im Referenzbild 11.2 das Abbild des Gasmessgeräts 1.2 durch die Markierung 21.2 gekennzeichnet. Die Bildvergleichseinheit 25 hat im Suchbild 31.2 ein Abbild des aufzufindenden Gasmessgeräts 1.2 erkannt. Das Suchgerät 4 zeigt auf dem Bildschirm 17 das Suchbild 31.2 an. Der Bildbereich, in dem das Gasmessgerät 1.2 gezeigt wird, ist durch die Markierung 33.2 hervorgehoben.

Möglich ist, dass die Bildvergleichseinheit 25 in keinem Suchbild 31.1, 31.2, 31.3, 31.4, 41.1, 41.2, 41.3, 41.4 ein Abbild des Gasmessgeräts 1.1, 1.2 gefunden hat. Figur 7 zeigt ein solches Beispiel. In diesem Beispiel soll das Gasmessgerät 1.1 aufgefunden werden. In der ersten Teilphase wurde der Servicetechniker zu einem Ort O.b mit der Geoposition Geo.b geführt. Der erste Teilphase verlief so wie weiter oben mit Bezug auf Figur 6 beschrieben. Das nunmehr aufzufindende Gasmessgerät 1.1 befindet sich im umschlossenen Raum 50.2, und die Tür 19 ist geschlossen, vgl. Figur 1 und Figur 2. Am Ort O.b erzeugt die Kamera 16 die Suchbilder 41.1, 41.2, 41.3, 41.4. Die Bildvergleichseinheit 25 findet in keinem Suchbild 41.1, 41.2, 41.3, 41.4 ein Abbild des Gasmessgeräts 1.1, welches im Referenzbild 11.1 gezeigt und durch die Markierung 21.1 hervorgehoben wird. Die Bildvergleichseinheit 25 ermittelt hingegen im Suchbild 41.2 ein Abbild eines Teils der Tür 19, wobei die Bildvergleichseinheit 25 das Referenzbild 11.1a und in diesem beispielsweise die Markierung XY und / oder eine besondere Form der Tür 19 detektiert. Das Suchgerät 4 zeigt auf dem Bildschirm 17 das Suchbild 41.2 an.

Möglich ist auch, dass der Servicetechniker eine Benutzereingabe macht, dass er das Gasmessgerät 1.2 nicht gefunden hat. In einer Realisierungsform wird der Servicetechnik gebeten, sich an einen anderen Ort zu begeben und erneut Suchbilder anzufertigen.

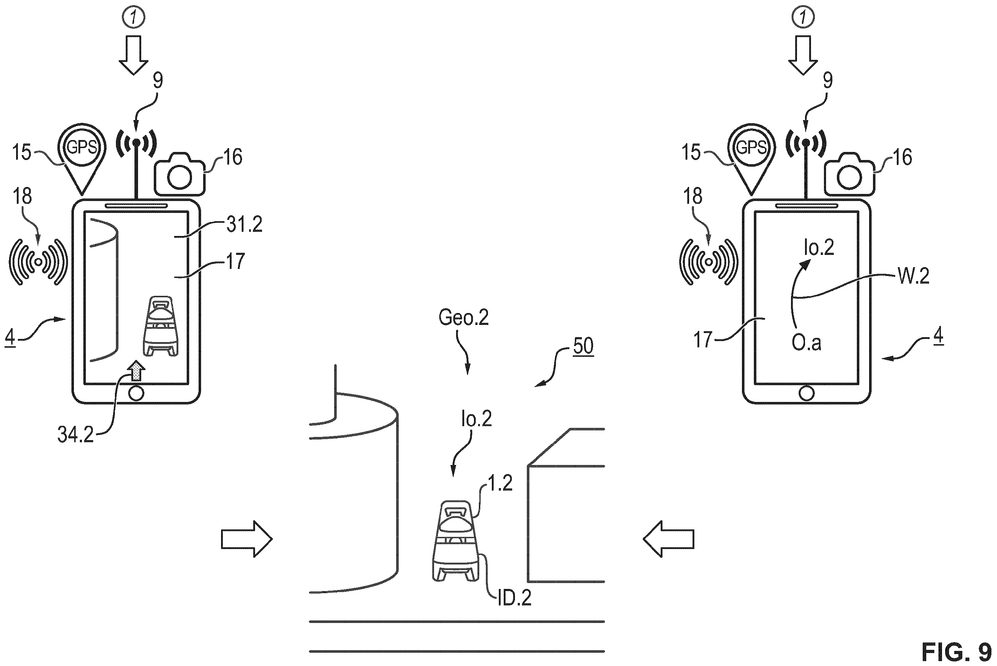

Figur 8 und Figur 9 zeigen eine alternative oder zusätzliche Realisierungsform. In diesem Beispiel soll wiederum das Gasmessgerät 1.2 gefunden werden. Gemäß dieser alternativen oder zusätzlichen Realisierungsform berechnet die Bildauswerteeinheit 25 einen Weg W.2 vom Ort O.a, an dem sich der Servicetechniker gerade befindet, zum gesuchten Installationsort Io.2 - oder wenigstens die Richtung vom Ort O.a zum Installationsort Io.2. Hierfür verwendet die Bildauswerteeinheit 25
- die gemessene Geoposition Geo.a des Orts O.a,
- die übermittelte Geoposition Geo.2 des Installationsorts Io.2,
- das Referenzbild 11.2,
- die Suchbilder 31.1, 31.2, 31.3, 31.4 und
- die jeweilige Blickrichtung R.1, R.2, R.3, R.4, aus der ein Suchbild 31.1, 31.2, 31.3, 31.4 erzeugt wurde.

Das Suchgerät 4 zeigt den berechneten Weg W.2 vom Ort O.a zum gesuchten Installationsort Io.2 auf dem Bildschirm 17 an. Im Beispiel von Figur 9 links wird auf dem Bildschirm 17 das Suchbild 31.2 gezeigt. In das Suchbild 31.2 ist ein Pfeil 34.2 eingeblendet, der die Richtung zum Installationsort Io.2 zeigt. Im Beispiel von Figur 9 rechts wird ein Pfeil gezeigt, der einen Weg W.2 vom Ort O.a zum Installationsort Io.2 spezifiziert. Dieser Pfeil kann beispielsweise in einen Ausschnitt aus einer Landkarte oder einen Grundriss der Produktionsanlage 50 (nicht gezeigt) eingeblendet sein.

Möglich ist, dass der Servicetechniker nunmehr das gesuchte Gasmessgerät 1.1, 1.2 findet. In einer Realisierungsform liest das optionale Lesegerät 18 die eindeutige Kennung ID.1, ID.2 des Gasmessgeräts 1.1, 1.2 ein. Dies ist eine Bestätigung dafür, dass der Servicetechniker das richtige Gasmessgerät 1.1, 1.2 gefunden hat. In einer anderen Realisierungsform liest der Servicetechniker eine Kennung ID.1, ID.2 in Form einer alphanumerischen Zeichenfolge auf einer Oberfläche des Gasmessgeräts 1.1, 1.2 und gibt eine Bestätigung ein, dass das gesuchte Gasmessgerät 1.1, 1.2 nunmehr gefunden ist.

Falls der Servicetechniker nicht das Gasmessgerät 1.1, 1.2 gefunden hat, wird bevorzugt die gerade beschriebene zweite Teilphase erneut durchgeführt. Vor der zweiten Teilphase befindet sich der Servicetechniker an einem Ort O.c. Die weiter oben beschriebenen Schritte werden erneut durchgeführt, diesmal mit dem Ort O.c anstelle des Orts O.a. Bei Bedarf wird die zweite Teilphase so oft wiederholt, bis der Servicetechniker das Gasmessgerät 1.1, 1.2 gefunden hat.

In einer Ausgestaltung wird mindestens einmal, bevorzugt mehrmals, ein Maß für den aktuellen Abstand zwischen einem aufzufindenden Gasmessgerät 1.x und dem Suchgerät 4 gemessen. Bevorzugt wird automatisch versucht, eine drahtlose Datenverbindung zwischen dem Gasmessgerät 1.x und dem Suchgerät 4 herzustellen. Die drahtlose Verbindung nutzt beispielsweise ein Protokoll gemäß dem Standard Bluetooth oder Bluetooth Low Energy (BLE) oder WLAN. Das Suchgerät 4 umfasst einen Empfänger für ein drahtlos übermitteltes Signal und bevorzugt auch einen Sender. Entsprechend umfasst das Gasmessgerät 1.x einen Sender und bevorzugt einen Empfänger. Ausreichend ist aber auch, dass das Gasmessgerät 1.x nur einen Sender und das Suchgerät 4 nur einen Empfänger umfasst.

Sobald diese Datenverbindung hergestellt ist, wird mindestens einmal das Maß für den Abstand gemessen. Um das Maß für den Abstand zu messen, wird beispielsweise die Stärke eines Signals gemessen, welches vom Gasmessgerät 1.x an der Suchgerät 4 übermittelt wird. Oder gemessen wird, wie lange ein Signal braucht, um vom Suchgerät 4 an das Gasmessgerät 1.x und zurück vom Gasmessgerät 1.x an das Suchgerät 4 übermittelt zu werden. Das oder ein Signal, welches vom Gasmessgerät 1.x zum Suchgerät 4 übermittelt wird, umfasst bevorzugt eine eindeutige Kennung des Gasmessgeräts 1.x, so dass das Suchgerät 4 automatisch entscheiden kann, welches Gasmessgerät sich in seiner Nähe befindet.

In einer bevorzugten Ausgestaltung gibt das Suchgerät 4 mindestens eine der folgenden Informationen aus, und zwar in einer von einem Menschen wahrnehmbaren Form, bevorzugt auf dem Bildschirm 17:
- das Ereignis, dass eine Datenverbindung zwischen dem Suchgerät 4 und dem aufzufindenden Gasmessgerät 1.x hergestellt ist,
- das Ereignis, dass diese Datenverbindung wieder unterbrochen ist,
- eine Kennzeichnung für den gemessenen Abstand dist,
- eine Kennzeichnung, ob der gemessene Abstand dist größer oder kleiner wird oder gleich bleibt.

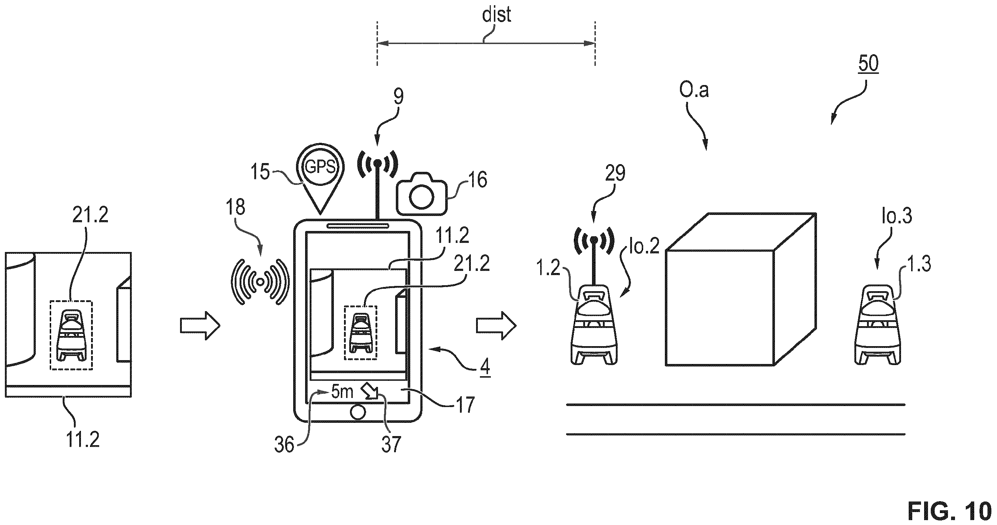

Figur 10 veranschaulicht beispielhaft diese Ausgestaltung. Das aufzufindende Gasmessgerät 1.2 umfasst eine Kommunikationseinheit mit einer Antenne 29. Wie bereits erwähnt, umfasst das Suchgerät 4 eine Kommunikationseinheit mit einer Antenne 9. Sobald eine drahtlose Datenverbindung zwischen dem Suchgerät 4 und dem Gasmessgerät 1.2 hergestellt ist, wird der Abstand dist gemessen. Im gezeigten Beispiel zeigt der Suchgerät 4 auf dem Bildschirm 17 folgende Informationen an:
- das übermittelte Referenzbild 11.2 des aufzufindenden Gasmessgeräts 1.2,
- eine Kennzeichnung 36 für den gemessenen Abstand dist (hier: 5 m) und
- eine Kennzeichnung 37, dass der Abstand dist kleiner geworden ist, der Servicetechniker sich also auf dem richtigen Weg befindet.

In einer Fortbildung dieser Ausgestaltung vermag das Suchgerät 4 zusätzlich zu ermitteln, in welche Richtung ein Pfad vom Suchgerät 4 zum Gasmessgerät 1.x verläuft. Bevorzugt vermag das Suchgerät 4 festzustellen, aus welcher Richtung ein Signal vom Gasmessgerät 1.x kommt. Das Suchgerät 4 umfasst mindestens zwei Empfänger für ein Signal vom Gasmessgerät 1.x. Die Richtung wird bevorzugt ebenfalls auf dem Bildschirm 17 angezeigt.

### Bezugszeichenliste

| | |
|---|---|
| 1.1, 1.2, 1.3 | stationäre Gasmessgeräte, an den Installationsorten Io.1, Io.2, Io.3 installiert, gehören zur Sensor-Anordnung des Ausführungsbeispiels |
| 3 | mobiles datenverarbeitendes Installationsgerät, beispielsweise Smartphone, umfasst die Kamera 6, den Geopositionssensor 5, das Lesegerät 8 und den Bildschirm 7 |
| 4 | mobiles datenverarbeitendes Suchgerät, beispielsweise Smartphone, umfasst die Kamera 16, den Geopositionssensor 15, das Lesegerät 18 und den Bildschirm 17 |
| 5 | Geopositionssensor des Installationsgeräts 3 |
| 6 | Kamera des Installationsgeräts 3, erzeugt die Referenzbilder 11.1, 11.1a, 11.2, 11.3 |
| 7 | berührungssensitiver Bildschirm des Installationsgeräts 3 |
| 8 | Lesegerät zum Lesen einer Kennung ID.1, ID.2, ID.3, gehört zum Installationsgerät 3 |
| 9 | Antenne, gehört zu einer Kommunikationseinheit des Suchgeräts 4 |
| 10.1, 10.2, 10.3 | Datensätze in der zentralen Datenbank 12 für die Gasmessgeräte 1.1, 1.2, 1.3, umfassen jeweils Informationen über die eindeutige Kennung ID.1, ID.2, ID.2, den Installationsort Io.1, Io.2, Io.3, den Zeitpunkt T.1, T.2, T.3 der letzten Wartung sowie das Referenzbild 11.1, 11.1a, 11.2, 11.3 |
| 11.1, 11.1a, 11.2, 11.3 | Referenzbilder von den Installationsorten Io.1, Io.2, Io.3, von der Kamera 6 erzeugt |
| 12 | zentrale Datenbank, in der für jedes Gasmessgerät 1.1, 1.2, 1.3 auf der Produktionsanlage 50 jeweils ein Datensatz 10.1, 10.2, 10.3 abgelegt ist |
| 13 | Zentralrechner, hat Lesezugriff und Schreibzugriff auf die zentrale Datenbank 12, empfängt Nachrichten vom Installationsgerät 3 |
| 15 | Geopositionssensor des Suchgeräts 4 |
| 16 | Kamera des Suchgeräts 4, erzeugt die Suchbilder 31.1, ..., 31.4, 41.1, ..., 41.4 |
| 17 | berührungssensitiver Bildschirm des Suchgeräts 4 |
| 18 | Lesegerät zum Lesen einer Kennung ID.1, ID.2, ID.3, gehört zum Suchgerät 4 |
| 19 | Tür zum umschlossenen Raum 50.2, trägt die Markierung XY |
| 21.1, 21.2, 21.3 | Markierungen, die den Bereich anzeigen, in dem im Referenzbilder 11.1, 11.2, 11.3 das Gasmessgerät 1.1, 1.2, 1.3 gezeigt wird |
| 25 | Bildvergleichseinheit, vergleicht automatisch die Suchbilder 31.1, 31.2, 31.3, 31.4 mit dem Referenzbild 11.2 |
| 26 | Bildauswerteeinheit, sucht in den Referenzbildern 11.1, 11.1a, 11.2, 11.3 nach einem Abbild eines Gasmessgeräts |
| 29 | Antenne, gehört zu einer Kommunikationseinheit des Gasmessgeräts 1.2 |
| 31.1, 31.2, 31.3, 31.4 | Suchbilder, bei der Suche nach dem Gasmessgerät 1.2 am Ort O.a vom Bildaufnahmegerät 16 angefertigt |
| 33.2 | Markierung des Bereichs, in dem das Suchbild 31.2 das Gasmessgerät 1.2 zeigt, wird zusammen mit dem Suchbild 31.2 auf dem Bildschirm 17 ausgegeben |
| 34.2 | Pfeil, der in das Suchbild 31.2 eingeblendet ist, um den Weg zum Installationsort Io.2 zu zeigen |
| 36 | Kennzeichnung des gemessenen Abstands dist zwischen dem Suchgerät 4 und dem aufzufindenden Gasmessgerät 1.2, wird auf dem Bildschirm 17 ausgegeben |
| 37 | Kennzeichnung, dass der Abstand dist zwischen den Suchgerät 4 und dem Gasmessgerät 1.2 kleiner wird, wird auf dem Bildschirm 17 ausgegeben |
| 41.1, 41.2, 41.3, 41.4 | Suchbilder, bei der Suche nach dem Gasmessgerät 1.1 am Ort O.a vom Bildaufnahmegerät 16 angefertigt |
| 50 | Produktionsanlage, in der die Gasmessgeräte 1.1, 1.2, 1.3 installiert sind, umfasst die Bestandteile 50.1, 50.2, 50.3, 50.4 |
| 50.1, 50.2, 50.3, 50.4 | Bestandteile der Produktionsanlage 50 |
| dist | gemessener Abstand zwischen dem Suchgerät 4 und dem aufzufindenden Gasmessgerät 1.2 |
| Geo.1, Geo.2, Geo.3 | Geopositionen der Installationsorte Io.1, Io.2, Io.3 |
| Geo.a | Geoposition des Orts O.a |
| Geo.b | Geoposition des Orts O.b |
| Geo.s | Geoposition des Ausgangspunkts Start |
| ID.1, ID.2, ID.3 | eindeutige Kennungen der Gasmessgeräte 1.1, 1.2, 1.3, sind in einer Ausgestaltung maschinenlesbar |
| Io.1, Io.2, Io.3 | Installationsorte, an denen die stationären Gasmessgeräte 1.1, 1.2, 1.3 installiert sind |
| T.1, T.2, T.3 | Zeitpunkt, an dem das Gasmessgerät 1.1, 1.2, 1.3 installiert oder zum letzten Mal gewartet wurde |
| O.a | Ort, zu dem der Servicetechniker geführt wird, indem die aktuelle Geoposition des Suchgeräts 4 mit der Geoposition Geo.2 verglichen wird, zugleich Ort, an dem die Suchbilder 31.1, 31.2, 31.3, 31.4 angefertigt werden, hat die Geoposition Geo.a |
| O.b | Ort, an dem die Suchbilder 41.1, 41.2, 41.3, 41.4 angefertigt werden, hat die Geoposition Geo.b |
| R.1, R.2, R.3, R.4 | Blickrichtungen, in die die Kamera 16 beim Erzeugen der Suchbilder 31.1, 31.2, 31.3, 31.4 gerichtet ist |
| Start | Ausgangspunkt, in dem der Installationstechniker die Suche nach dem Installationsort Io.2 beginnt, hat die Geoposition Geo.s |
| W.1 | Weg vom Ausgangspunkt Start zum Installationsort Io.2, vom Suchgerät 4 aufgrund der Geopositionen Geo.a und Geo.2 berechnet, wird auf dem Bildschirm 17 angezeigt |
| W.2 | Weg vom Ort O.a zum Installationsort Io.2, vom Suchgerät 4 aufgrund der Suchbilder 31.1, 31.2, 31.3, 31.4 und des Referenzbilds 11.2 berechnet, wird auf dem Bildschirm 17 angezeigt |

## Patentansprüche

1. Betriebs-Verfahren zum Betreiben einer Sensor-Anordnung,
wobei die Sensor-Anordnung mindestens einen Sensor (1.1, 1.2, 1.3) umfasst, bevorzugt mehrere Sensoren,
wobei der oder jeder Sensor (1.1, 1.2, 1.3) der Sensor-Anordnung
- jeweils dazu ausgestaltet ist, eine physikalische Größe zu messen und / oder zu prüfen, ob die physikalischen Größe in einem vorgegebenen Wertebereich liegt oder nicht, und
- an jeweils einem Installationsort (Io.1, Io.2, Io.3) installiert wird oder ist,
wobei das Betriebs-Verfahren unter Verwendung einer Betriebs-Anordnung durchgeführt wird,
wobei die Betriebs-Anordnung
- ein mobiles datenverarbeitendes Installationsgerät (3),
- ein mobiles datenverarbeitendes Suchgerät (4),
- einen Zentralrechner (13) und
- eine zentrale Datenbank (12)
umfasst,
wobei das Installationsgerät (3) ein Bildaufnahmegerät (6) umfasst,
wobei das Betriebs-Verfahren eine Installationsphase und mindestens eine nachfolgende Suchphase umfasst,
wobei die Installationsphase für jeden Sensor (1.1, 1.2, 1.3) der Sensor-Anordnung durchgeführt wird und jeweils die Schritte umfasst, dass
- das Bildaufnahmegerät (6) des Installationsgeräts (3) mindestens ein Referenzbild (11.1, 11.1a, 11.2, 11.3) vom Installationsort (Io.1, Io.2, Io.3) des Sensors (1.1, 1.2, 1.3) erzeugt,
- das oder jedes Referenzbild (11.1, 11.1a, 11.2, 11.3) vom Installationsgerät (3) an den Zentralrechner (13) übermittelt wird und
- der Zentralrechner (13) bewirkt, dass in der zentralen Datenbank (12) ein Datensatz (10.1, 10.2, 10.3) für den Sensor (1.1, 1.2, 1.3) angelegt oder ein bereits vorhandener Datensatz ergänzt wird,
wobei der Datensatz (10.1, 10.2, 10.3) nach dem Anlegen oder Ergänzen das oder jedes übermittelte Referenzbild (11.1, 11.1a, 11.2, 11.3) vom Installationsort (Io.1, Io.2, Io.3) des Sensors (1.1, 1.2, 1.3) umfasst, und
wobei die Suchphase für mindestens einen aufzufindenden Sensor (1.1, 1.2) der Sensor-Anordnung durchgeführt wird und
wobei die Suchphase für den oder jeden aufzufindenden Sensor (1.1, 1.2) jeweils die Schritte umfasst, dass
- das oder jedes Referenzbild (11.1, 11.1a, 11.2), das vom Datensatz (10.1, 10.2, 10.3) des aufzufindenden Sensors (1.1, 1.2) umfasst wird, ermittelt wird,
- unter Verwendung des oder mindestens eines, bevorzugt jedes ermittelten Referenzbilds (11.1, 11.1a, 11.2) eine Information (33.2, 34.2, W.2) über den Installationsort (Io.1, Io.2) ermittelt wird und
das Suchgerät (4) die ermittelte Installationsort-Information (33.2, 34.2, W.2) in mindestens einer von einem Menschen wahrnehmbaren Form ausgibt.

2. Betriebs-Verfahren nach Anspruch 1,
**dadurch gekennzeichnet, dass**
in der Installationsphase mindestens eines Sensors (1.1) mindestens zwei Referenzbilder (11.1, 11.1a) vom Installationsort (lo.1) erzeugt werden,
wobei das erste Referenzbild (11.1) den Sensor (1.1) zeigt und
wobei das zweite Referenzbild (11.1a) einen Gegenstand (50.2, 19) zeigt, der sich beim Erzeugen des zweiten Referenzbilds (11.1a) zwischen dem Bildaufnahmegerät (6) des Installationsgeräts (3) und dem Sensor (1.1) befindet und daher den Sensor (1.1) vollständig oder wenigstens teilweise verdeckt.

3. Betriebs-Verfahren nach einem der vorhergehenden Ansprüche,
**dadurch gekennzeichnet, dass**
die Betriebs-Anordnung zusätzlich eine signalverarbeitende Bildauswerteeinheit (26) umfasst und
die Installationsphase für jeden Sensor (1.1, 1.2, 1.3) der Sensor-Anordnung zusätzlich die Schritte umfasst, dass
- die Bildauswerteeinheit (26) jedes Referenzbild (11.1, 11.1a, 11.2, 11.3) vom Installationsort (Io.1, Io.2, Io.3) des Sensors (1.1, 1.2, 1.3) nach einem Abbild eines Sensors (1.1, 1.2, 1.3) durchsucht,
- dann, wenn die Bildauswerteeinheit (26) in jedem Referenzbild (11.1, 11.1a, 11.2, 11.3) vom Installationsort (Io.1, Io.2, Io.3) jeweils entweder überhaupt kein Abbild eines Sensors (1.1, 1.2, 1.3) oder zwei Abbilder von zwei verschiedenen Sensoren findet,
die Bildauswerteeinheit (26) bewirkt, dass eine entsprechenden Nachricht generiert wird, und
- das Installationsgerät (4) diese Nachricht in einer von einem Menschen wahrnehmbaren Form ausgibt.

4. Betriebs-Verfahren nach einem der vorhergehenden Ansprüche,
**dadurch gekennzeichnet, dass**
die ermittelte Installationsort-Information (33.2, 34.2, W.2) das oder jedes vom Datensatz (10.1, 10.2, 10.3) umfasste und in der Suchphase ermittelte Referenzbild (11.1, 11.1a, 11.2) umfasst und
der Schritt, für einen aufzufindenden Sensor (1.1, 1.2) die ermittelte Installationsort-Information (33.2, 34.2, W.2) auszugeben, die Schritte umfasst, dass
das oder jedes ermittelte Referenzbild (11.1, 11.1a, 11.2, 11.3) des Sensors (1.1, 1.2) an das Suchgerät (4) übermittelt und vom Suchgerät (4) ausgegeben wird.

5. Betriebs-Verfahren nach einem der vorhergehenden Ansprüche,
**dadurch gekennzeichnet, dass**
auch das Suchgerät (4) ein Bildaufnahmegerät (16) umfasst und
die Suchphase für den oder mindestens einen, bevorzugt für jeden aufzufindenden Sensor (1.1, 1.2) jeweils den zusätzlichen Schritt umfasst, dass
das Bildaufnahmegerät (16) des Suchgeräts (4) mindestens ein Suchbild, bevorzugt mehrere Suchbilder (31.1, 31.2, 31.3, 31.4, 41.1, 41.2, 41.3, 41.4), von einer Umgebung des Suchgeräts (4) erzeugt.

6. Betriebs-Verfahren nach Anspruch 5,
**dadurch gekennzeichnet, dass**
sowohl das Installationsgerät (3) als auch das Suchgerät (4) zusätzlich jeweils einen Geopositionssensor (5, 15) umfasst,
die Installationsphase für jeden Sensor (1.1, 1.2, 1.3) der Sensor-Anordnung jeweils die zusätzlichen Schritte umfasst, dass
- der Geopositionssensor (5) des Installationsgeräts (3) eine Geoposition (Geo.1, Geo.2, Geo.3) des Installationsorts (Io.1, Io.2, Io.3) misst,
- die gemessene Geoposition (Geo.1, Geo.2, Geo.3) des Installationsorts (Io.1, Io.2, Io.3) vom Installationsgerät (3) an den Zentralrechner (13) übermittelt wird und
- der Zentralrechner (13) bewirkt, dass der Datensatz (10.1, 10.2, 10.3), der für den Sensor (1.1, 1.2, 1.3) angelegt oder ergänzt wird, zusätzlich die übermittelte Geoposition (Geo.1, Geo.2, Geo.3) des Installationsorts (Io.1, Io.2, Io.3) des Sensors (1.1, 1.2, 1.3) umfasst,
die Suchphase für den oder jeden aufzufindenden Sensor (1.1, 1.2) den zusätzlichen Schritt umfasst, dass
der Geopositionssensor (15) des Suchgeräts (4) mindestens einmal seine eigene Geoposition (Geo.a, Geo.b) misst, während das oder ein Suchbild (31.1, 31.2, 31.3, 31.4, 41.1, 41.2, 41.3, 41.4) erzeugt wird, und
das Suchgerät (4) für die Ermittlung der Installationsort-Information (33.2, 34.2, W.2) zusätzlich
- die in der Installationsphase gemessene und im Datensatz (10.1, 10.2) abgespeicherte Geoposition (Geo.1, Geo.2) des Installationsorts (Io.1, Io.2) und
- die oder jede Geoposition (Geo.a, Geo.b), die der Geopositionssensor (15) des Suchgeräts (4) bei der Erzeugung der Suchbilder (31.1, 31.2, 31.3, 31.4, 41.1, 41.2, 41.3, 41.4) gemessen hat,
verwendet.

7. Betriebs-Verfahren nach Anspruch 5 oder Anspruch 6,
**dadurch gekennzeichnet, dass**
die Betriebs-Anordnung eine signalverarbeitende Bildvergleichseinheit (25) umfasst,
wobei die Suchphase für den oder mindestens einen, bevorzugt für jeden aufzufindenden Sensor (1.1, 1.2) jeweils die zusätzlichen Schritte umfasst, dass
- das oder jedes vom Datensatz (10.1, 10.2, 10.3) des aufzufindenden Sensors (1.1, 1.2) umfasste Referenzbild (11.1, 11.1a, 11.2) und das oder jedes in der Suchphase erzeugte Suchbild (31.1, 31.2, 31.3, 31.4, 41.1, 41.2, 41.3, 41.4) des Sensors (1.1, 1.2) an die Bildvergleichseinheit (25) übermittelt werden,
- die Bildvergleichseinheit (25) durch einen rechnerischen Bildvergleich von übermittelten Bildern
in mindestens einem Suchbild (31.1, 31.2, 31.3, 31.4, 41.1, 41.2, 41.3, 41.4) den Installationsort (Io.1, Io.2) des Sensors (1.1, 1.2) ermittelt oder feststellt, dass kein Suchbild (31.1, 31.2, 31.3, 31.4, 41.1, 41.2, 41.3, 41.4) den Installationsort (Io.1, Io.2) des Sensors (1.1, 1.2) zeigt,
- das Ergebnis des Bildvergleichs an das Suchgerät (4) übermittelt wird und
- dann, wenn die Bildvergleichseinheit (25) in mindestens einem Suchbild (31.1, 31.2, 31.3, 31.4, 41.1, 41.2, 41.3, 41.4) den Installationsort (lo.1, Io.2) ermittelt hat,
das Suchgerät (4) die Information (33.2, 34.2, W.2) über den Installationsort (Io.1, Io.2) unter Verwendung des Ergebnisses des Bildvergleichs ermittelt.

8. Betriebs-Verfahren nach Anspruch 7,
**dadurch gekennzeichnet, dass**
das Betriebs-Verfahren den zusätzlichen Schritt umfasst, dass
die Bildvergleichseinheit (25) in den Suchbildern (31.1, 31.2, 31.3, 31.4, 41.1, 41.2, 41.3, 41.4) nach einem Abbild des aufzufindenden Sensors (1.2) sucht,
wobei die Bildvergleichseinheit (25) für die Suche nach dem Abbild das oder mindestens ein Referenzbild (11.1, 11.1a, 11.2) vom Installationsort (Io.1, Io.2) des aufzufindenden Sensors (1.1, 1.2) verwendet, und
dann, wenn mindestens ein solches Suchbild (31.2) mit einem Abbild des Sensors (1.2) gefunden ist,
der Schritt, dass das Suchgerät (4) die Installationsort-Information (33.2, 34.2, W.2) ausgibt, den Schritt umfasst, dass
das Suchgerät (4) das oder mindestens ein Suchbild (31.2), welches den Sensor (1.2) zeigt, zusammen mit einer Markierung (33.2) eines Bildbereichs, in dem im Suchbild (31.2) das Abbild des aufzufindenden Sensors (1.2) gezeigt wird, ausgibt.

9. Betriebs-Verfahren nach Anspruch 6,
**dadurch gekennzeichnet, dass**
die Suchphase für mindestens einen aufzufindenden Sensor (1.1, 1.2) eine erste Teilphase und eine nachfolgende zweite Teilphase umfasst,
wobei die Schritte, dass
- das oder jedes Referenzbild (11.1, 11.1a, 11.2), das vom Datensatz (10.1, 10.2, 10.3) des aufzufindenden Sensors (1.1, 1.2) umfasst wird, ermittelt wird,
- unter Verwendung des oder jedes ermittelten Referenzbilds (11.1, 11.1a, 11.2) eine Information (33.2, 34.2, W.2) über den Installationsort (Io.1, Io.2) ermittelt wird und
das Suchgerät (4) die ermittelte Installationsort-Information (33.2, 34.2, W.2) ausgibt,
in der zweiten Teilphase durchgeführt werden und
die erste Teilphase die Schritte umfasst, dass
- die abgespeicherte Geoposition (Geo.1, Geo.2) des Installationsorts (Io.1, Io.2) des aufzufindenden Sensors (1.1, 1.2) an das Suchgerät (4) übermittelt wird,
- der Geopositionssensor (15) des Suchgeräts (4) mindestens einmal die eigene Geoposition (Geo.s) misst, während das Suchbild (31.1, 31.2, 31.3, 31.4, 41.1, 41.2, 41.3, 41.4) erzeugt wird,
- das Suchgerät (4) einen Weg (W.1) von der gemessenen eigenen Geoposition (Geo.s) zur übermittelten Geoposition (Geo.1, Geo.2) ermittelt und
- das Suchgerät (4) eine Information über den ermittelten Weg (W.1) in einer von einem Menschen wahrnehmbaren Form ausgibt,
wobei das Suchgerät (4) für die Ermittlung des Wegs (W.1)
- die im Datensatz (10.1, 10.2) abgespeicherte übermittelte Geoposition (Geo.1, Geo.2) und
- die oder jede Geoposition (Geo.s), die der Geopositionssensor (15) des Suchgeräts (4) in der ersten Teilphase misst,
verwendet.

10. Betriebs-Verfahren nach Anspruch 7 oder nach Anspruch 8 und nach Anspruch 9,
**dadurch gekennzeichnet, dass** die Schritte, dass
- das Bildaufnahmegerät (16) mindestens ein Suchbild (31.1, 31.2, 31.3, 31.4, 41.1, 41.2, 41.3, 41.4) erzeugt,
- das oder jedes Referenzbild (11.1, 11.1a, 11.2) für den aufzufindenden Sensor (1.1, 1.2) und das oder jedes Suchbild (31.1, 31.2, 31.3, 31.4, 41.1, 41.2, 41.3, 41.4) an die Bildvergleichseinheit (25) übermittelt werden,
- die Bildvergleichseinheit (25) in den Suchbildern (31.1, 31.2, 31.3, 31.4, 41.1, 41.2, 41.3, 41.4) den Installationsort (Io.1, Io.2) ermittelt oder feststellt, dass kein Suchbild (31.1, 31.2, 31.3, 31.4, 41.1, 41.2, 41.3, 41.4) den Installationsort (Io.1, Io.2) zeigt,
- das Ergebnis des Bildvergleichs an das Suchgerät (4) übermittelt wird und
- das Suchgerät (4) unter Verwendung des Bildvergleichs-Ergebnisses die Installations-Information (33.2, 34.2, W.2) ermittelt und ausgibt,
in der zweiten Teilphase durchgeführt werden.

11. Betriebs-Verfahren nach Anspruch 9 oder Anspruch 10,
**dadurch gekennzeichnet, dass**
das Suchgerät (4) nach der ersten Teilphase eine Benutzereingabe erfasst, ob der aufzufindende Sensor (1.1, 1.2) nach der ersten Teilphase gefunden worden ist oder nicht, und
die zweite Teilphase nur dann durchgeführt wird, wenn gemäß der Benutzereingabe der Sensor (1.1, 1.2) nach der ersten Teilphase nicht aufgefunden worden ist.

12. Betriebs-Verfahren nach einem der Ansprüche 7 bis 11,
**dadurch gekennzeichnet, dass**
der Schritt, dass das Suchgerät (4) die Installationsort-Information (33.2, 34.2, W.2) ermittelt, den Schritt umfasst, dass
die Bildvergleichseinheit (25)
- die Suchbilder (31.1, 31.2, 31.3, 31.4, 41.1, 41.2, 41.3, 41.4) mit dem oder einem, bevorzugt mit jedem Referenzbild (11.1, 11.1a, 11.2) des aufzufindenden Sensors (1.1, 1.2) vergleicht und
- jeweils ein Übereinstimmungs-Maß für die Übereinstimmung zwischen einem Suchbild (31.1, 31.2, 31.3, 31.4, 41.1, 41.2, 41.3, 41.4) und einem Referenzbild (21.2) berechnet, und
der Schritt, dass das Suchgerät (4) die Installationsort-Information (33.2, 34.2, W.2) ausgibt, den Schritt umfasst, dass
das Suchgerät (4) das oder mindestens ein Suchbild (31.2) ausgibt, welches mit dem oder mindestens einem Referenzbild (11.2) das größte Übereinstimmungs-Maß und / oder ein Übereinstimmungs-Maß oberhalb einer vorgegebenen unteren Schranke aufweist.

13. Betriebs-Verfahren nach einem der Ansprüche 7 bis 12,
**dadurch gekennzeichnet, dass**
die Erzeugung mindestens eines Suchbilds (31.1, 31.2, 31.3, 31.4) den zusätzlichen Schritt umfasst, dass
das Suchgerät (4) eine Blickrichtung (R.1, R.2, R.3, R.4), in welcher das Bildaufnahmegerät (16) das Suchbild (31.1, 31.2, 31.3, 31.4) erzeugt, ermittelt, und
der Schritt, dass das Suchgerät (4) die Installationsort-Information (33.2, 34.2, W.2) ermittelt, den Schritt umfasst, dass
das Suchgerät (4) einen Weg (W.2) von dem Ort (O.a), an dem mindestens ein Suchbild (31.1, 31.2, 31.3, 31.4) erzeugt worden ist, zum Installationsort (Io.2) des aufzufindenden Sensors (1.2) ermittelt,
wobei das Suchgerät (4) für die Ermittlung des Wegs (W.2) zum Installationsort (lo.2)
- das Ergebnis des Bildvergleichs und
- die ermittelten Blickrichtungen (R.1, R.2, R.3, R.4) verwendet, und
der Schritt, dass das Suchgerät (4) die Installationsort-Information (33.2, 34.2, W.2) ausgibt, den Schritt umfasst, dass
das Suchgerät (4) eine Information über den berechneten Weg (W.2) in mindestens einer von einem Menschen wahrnehmbaren Form ausgibt.

14. Betriebs-Verfahren nach einem der vorhergehenden Ansprüche,
**dadurch gekennzeichnet, dass**
der oder mindestens ein Sensor der Sensor-Anordnung an einem Installationsort installiert wird oder ist, der sich in einem Gebäude mit mehreren Stockwerken befindet,
wobei der Schritt, dass das Bildaufnahmegerät (6) des Installationsgeräts (3) mindestens ein Referenzbild vom Installationsort des Sensors erzeugt, den Schritt umfasst, dass
das Bildaufnahmegerät (3) mindestens ein Referenzbild erzeugt, welches eine Kennzeichnung des Stockwerks, in dem sich der Installationsort befindet, umfasst.

15. Betriebs-Verfahren nach einem der vorhergehenden Ansprüche,
**dadurch gekennzeichnet, dass**
die Suchphase für den oder mindestens einen aufzufindenden Sensor (1.2) die zusätzlichen Schritte umfasst, dass
- mindestens einmal der aktuelle Abstand (dist) zwischen dem Sensor (1.2) und dem Suchgerät (4) gemessen wird und
- das Suchgerät (4) eine Kennzeichnung für den gemessenen aktuellen Abstand (dist) und / oder eine Kennzeichnung, ob der gemessene Abstand (dist) größer oder kleiner wird oder gleich bleibt, ausgibt.

16. Betriebs-Verfahren nach einem der vorhergehenden Ansprüche,
**dadurch gekennzeichnet, dass**
der oder mindestens ein Sensor (1.1, 1.2, 1.3) der Sensor-Anordnung ein Gasmessgerät ist,
wobei das oder jedes Gasmessgerät (1.1, 1.2, 1.3) der Sensor-Anordnung jeweils dazu ausgestaltet ist,
mindestens ein vorgegebenes Zielgas zu detektieren und / oder die Konzentration mindestens eines Zielgases zu messen.

17. Betriebs-Verfahren nach einem der vorhergehenden Ansprüche,
**dadurch gekennzeichnet, dass**
der oder mindestens ein Sensor (1.1, 1.2, 1.3) der Sensor-Anordnung eine Kommunikationseinheit umfasst,
wobei die Kommunikationseinheit dazu ausgestaltet ist,
- eine Nachricht zu erzeugen und
- zu veranlassen, dass die Nachricht an einen räumlich entfernten Empfänger übermittelt wird, und
wobei die Nachricht eine Information über ein Messergebnis des Sensors (1.1, 1.2, 1.3) umfasst.

18. Betriebs-Anordnung zum Betreiben einer Sensor-Anordnung,
wobei die Sensor-Anordnung mindestens einen Sensor (1.1, 1.2, 1.3) umfasst, bevorzugt mehrere Sensoren,
wobei der oder jeder Sensor (1.1, 1.2, 1.3) der Sensor-Anordnung
- jeweils dazu ausgestaltet ist, eine physikalische Größe zu messen und / oder zu prüfen, ob die physikalischen Größe in einem vorgegebenen Wertebereich liegt oder nicht, und
- an jeweils einem Installationsort (Io.1, Io.2, Io.3) installiert oder installierbar ist,
wobei die Betriebs-Anordnung
- ein mobiles datenverarbeitendes Installationsgerät (3),
- ein mobiles datenverarbeitendes Suchgerät (4),
- einen Zentralrechner (13) und
- eine zentrale Datenbank (12)
umfasst,
wobei das Installationsgerät (3) ein Bildaufnahmegerät (6) umfasst, welches dazu ausgestaltet ist, Bilder zu erzeugen,
wobei wenigstens zeitweise eine Datenverbindung vom Installationsgerät (3) zum Zentralrechner (13) hergestellt ist,
wobei der Zentralrechner (13) dazu ausgestaltet ist, in der zentralen Datenbank (12) für jeden Sensor (1.1, 1.2, 1.3) der Sensor-Anordnung jeweils einen Datensatz (10.1, 10.2, 10.3) anzulegen,
wobei der Datensatz (10.1, 10.2, 10.3) für einen Sensor (1.1, 1.2, 1.3) mindestens ein Referenzbild (11.1, 11.1a, 11.2, 11.3) umfasst, das ist ein Bild, das den Installationsort (Io.1, Io.2, Io.3) des Sensors (1.1, 1.2, 1.3) zeigt und vom Installationsgerät (3) erzeugt worden ist,
wobei mindestens ein installierter Sensor (1.1, 1.2) der Sensor-Anordnung ein aufzufindender Sensor ist oder sein kann,
wobei die Betriebs-Anordnung dazu ausgestaltet ist,
- das oder jedes vom Datensatz (10.1, 10.2, 10.3) des oder eines aufzufindenden Sensors (1.1, 1.2) umfasste Referenzbild (11.1, 11.1a, 11.2) zu ermitteln und
- unter Verwendung des oder jedes ermittelten Referenzbilds (11.1, 11.1a, 11.2) eine Information (33.2, 34.2, W.2) über den Installationsort (Io.1, Io.2) zu ermitteln, und
wobei das Suchgerät (4) dazu ausgestaltet ist, die Installationsort-Information (33.2, 34.2, W.2) in mindestens einer von einem Menschen wahrnehmbaren Form auszugeben.

19. Betriebs-Anordnung nach Anspruch 18,
**dadurch gekennzeichnet, dass**
die Betriebs-Anordnung eine signalverarbeitende Bildvergleichseinheit (25) umfasst und
auch das Suchgerät (4) ein Bildaufnahmegerät (16) umfasst, welches dazu ausgestaltet ist, Bilder zu erzeugen,
wobei die Bildvergleichseinheit (25)
- ein Bestandteil des Suchgeräts (4) ist oder
- wenigstens zeitweise in einer bidirektionalen Datenverbindung mit dem Suchgerät (4) steht,
wobei die Bildvergleichseinheit (25) dazu ausgestaltet ist, automatisch
- für einen Sensor (1.1, 1.2) der Sensor-Anordnung ein in der Datenbank (12) abgespeichertes Referenzbild (11.1, 11.1a, 11.2) des Sensors (1.1, 1.2) mit mindestens einem Suchbild (31.1, 31.2, 31.3, 31.4, 41.1, 41.2, 41.3, 41.4), das ist ein vom Bildaufnahmegerät (16) des Suchgeräts (4) erzeugtes Bild, zu vergleichen und
- durch diesen Bildvergleich in dem Suchbild (31.1, 31.2, 31.3, 31.4, 41.1, 41.2, 41.3, 41.4) den Installationsort (Io.1, Io.2) des Sensors (1.1, 1.2) zu ermitteln oder festzustellen, dass kein Suchbild (31.1, 31.2, 31.3, 31.4, 41.1, 41.2, 41.3, 41.4) den Installationsort (Io.1, Io.2) zeigt,
wobei die Betriebs-Anordnung dazu ausgestaltet ist, für einen vorgegebenen Sensor (1.1, 1.2) der Sensor-Anordnung automatisch
dann, wenn in mindestens einem Suchbild (31.1, 31.2, 31.3, 31.4, 41.1, 41.2, 41.3, 41.4) der Installationsort (Io.1, Io.2) ermittelt ist,
eine Information (33.2, 34.2, W.2) über den Installationsort (Io.1, Io.2) des Sensors (1.1, 1.2) zu ermitteln, und
wobei die Betriebs-Anordnung weiterhin dazu ausgestaltet ist,
bei der Ermittlung der Installationsort-Information (33.2, 34.2, W.2) den durch den Bildvergleich ermittelten Installationsort (Io.1, Io.2) zu verwenden.

20. System umfassend
- eine Betriebs-Anordnung nach Anspruch 18 oder Anspruch 19 und
- eine Sensor-Anordnung,
wobei die Sensor-Anordnung mindestens einen Sensor (1.1, 1.2, 1.3) umfasst, bevorzugt mehrere Sensoren,
wobei der oder jeder Sensor (1.1, 1.2, 1.3) der Sensor-Anordnung
- jeweils dazu ausgestaltet ist, eine physikalische Größe zu messen und / oder zu prüfen, ob die physikalische Größe in einem vorgegebenen Wertebereich liegt oder nicht, und
- an jeweils einem Installationsort (Io.1, Io.2, Io.3) installiert ist und
wobei die Betriebs-Anordnung zum Betreiben der Sensor-Anordnung ausgestaltet ist.
